# EUROPEAN PATENT SPECIFICATION

(11) **EP 3 738 601 B1**
(45) Date of publication and mention of the grant of the patent: **25.11.2026**
(21) Application number: 18848077.6
(22) Date of filing: 23.08.2018
(51) Int. Cl.: A61K 38/10, A01K 67/027, A61K 47/66, A61P 15/00, A61P 35/00, A61P 43/00, C07K 7/08, C07K 19/00

(54) **CYTOCIDAL AGENT**
ZELLZERSTÖRENDES MITTEL
AGENT CYTOCIDE

(30) Priority: 24.08.2017 JP 2017161556
(43) Date of publication of application: 18.11.2020
(73) Proprietor: Fujita Academy, Toyoake-shi, Aichi 470-1192 (JP)
(72) Inventor: SUGIHARA Kazuhiro, Hamamatsu-shi Shizuoka 431-3192 (JP); KANAYAMA Naohiro, Hamamatsu-shi Shizuoka 431-3192 (JP); ONODERA Yuichiro, Hamamatsu-shi Shizuoka 431-3192 (JP); SHIBATA Toshiaki, Hamamatsu-shi Shizuoka 431-3192 (JP); FUKUDA Michiko, Tsukuba-shi Ibaraki 305-8560 (JP); NONAKA Motohiro, Tsukuba-shi Ibaraki 305-8560 (JP)
(74) Representative: Cabinet Becker et Associés
(86) International application number: PCT/JP2018/031136
(87) International publication number: WO 2019/039540

(56) References cited:
- WO-A1-2011/027312
- WO-A1-2011/079304
- WO-A1-2015/041019
- WO-A2-2007/076501
- WO-A2-2014/201118
- JP-A- 2013 523 824
- US-A1- 2016 145 308
- S. HATAKEYAMA ET AL: "Targeted drug delivery to tumor vasculature by a carbohydrate mimetic peptide", PROCEEDINGS OF THE NATIONAL ACADEMY OF SCIENCES, vol. 108, no. 49, 6 December 2011 (2011-12-06), pages 19587 - 19592, XP055040103, ISSN: 0027-8424, DOI: 10.1073/pnas.1105057108
- K. SUGIHARA ET AL: "Development of pro-apoptotic peptides as potential therapy for peritoneal endometriosis", NATURE COMMUNICATIONS, vol. 5, 22 June 2014 (2014-06-22), GB, XP055255332, ISSN: 2041-1723, DOI: 10.1038/ncomms5478
- SUGIHARA, KAZUHIRO ET AL: "Search of peptide therapeutic drugs for endometriosis", PROGRAMS AND ABSTRACTS OF THE 32ND ANNUAL MEETING OF THE JAPAN SOCIETY OF DRUG DELIVERY SYSTEM, 2016, pages 170, XP009519434
- SHIBATA, TOSHIAKI ET AL: "P3-14-1 Anti-tumor drugs using peptide that mimics sugar chains", ACTA OBSTETRICA ET GYNAECOLOGICA JAPONICA, vol. 68, no. 2, 2016, pages 858, XP009519433, ISSN: 0300-9165
- SUGIHARA, K. ET AL.: "Development of pro-apoptotic peptides as potential therapy for peritoneal endometriosis", NATURE COMMUNICATIONS, vol. 5, no. 4478, 2014, pages 1 - 9, XP055255332, ISSN: 2041-1723, DOI: 10.1038/ncomms5478

## Description

### TECHNICAL FIELD

The present invention relates to a cytocidal agent and it use for the treatment of a disease resulting from abnormal proliferation of a cell, wherein the disease is endometriosis or cancer..

### BACKGROUND ART

Apoptosis means cell death controlled and adjusted as a proliferation inhibition mechanism among cell deaths of multicellular organisms. In multicellular organisms, exclusion of unnecessary cells or harmful cells caused in a generation or reproduction process is performed by inducing apoptosis in the cell. In addition, by inducing apoptosis in a cell which is a cause of a disease and excluding the cell, it is possible to expect improvement in condition of the disease. In this way, substances having activity of inducing apoptosis is used for therapeutic use. For example, by inducing apoptosis in a cancer cell, it can be expected to remit or cure cancer.

In a case where apoptosis induction is used for treatment of a disease, selectivity with respect to a target cell in which apoptosis is induced is very important. In a case where target selectivity is low and apoptosis is induced in a cell other than a target cell, side effects exceed expected therapeutic effects and are not appropriate as a therapeutic drug from a viewpoint of safety. If apoptosis can be induced specifically in a target cell, an effective therapeutic drug in which side effects are sufficiently suppressed is obtained.

Endometriosis is a disease in which endometrial cells proliferate at a site other than an endometrial cavity. In many cases, endometrial cells proliferate in a peritoneum or ovary of pelvis. Representative symptoms include menstrual pain (dysmenorrhea) or infertility, and in a critical case, a patient can experience severe pain or be fainted. In addition, with endometriosis being a matrix, there is a case where cancer occurs. Endometrial cells proliferate under the stimulus of female hormones (estrogen). For this reason, as a therapeutic drug of endometriosis, hormone drugs for inhibiting estrogen secretion, for example, a low-dose pill, a selective agonist (inhibitor against ovarian function and endometrial cell proliferation), and a GnRH agonist (secretion inhibitor of follicle-stimulating hormone) are used. Here, since curative therapy has only an operation of removing the uterus and ovary, it is required to develop a therapeutic drug with which a cure of endometriosis is expected.

For example, Patent Literature 1 discloses a peptide composition including a fusion peptide between a Z13 peptide and an endosome escape peptide specifically binding to cyclic nucleotide-gated channel beta 3 (CNGB3) and a fusion peptide between a Z13 peptide and an apoptosis-inducing peptide. CNGB3 is a molecule that is specifically highly expressed on a cell surface of an endometrial cell (endometrial cell present other than endometriosis) and is not expressed on a peritoneum surface. By incorporating both peptides in an endometrial cell by the Z13 peptide moiety, it is possible to selectively induce apoptosis in an endometrial cell. In one example, a peptide composition including a fusion peptide between a Z13 peptide and an endosome escape peptide and a fusion peptide between a Z13 peptide and an apoptosis-inducing peptide was administered to the peritoneum of a baboon which has developed endometriosis via laparoscopy, selective apoptosis was induced only in a cell of a lesion of endometriosis, and apoptosis was not induced in other adjacent cells (refer to NPL 1).

### Citation List

### Patent Literature

[PTL 1] United States Patent Application, Publication No. 2016/145308

### Non-Patent Literature

[NPL 1] Sugihara, et al., NATURE COMMUNICATIONS, 2014, Volume 5, Article Number 4478.

### DISCLOSURE OF INVENTION

### Technical Problem

An object of the present disclosure is to provide a peptide drug that selectively induces apoptosis in a target cell.

### Solution to Problem

The present inventors found that, as a result of intensive examination to solve the problem, a peptide composition including a fusion peptide between an endosome escape peptide including a specific peptide sequence, an apoptosis-inducing peptide including a specific peptide sequence, and a Z13 peptide has a high efficiency in terms of higher selectivity and can induce apoptosis in a endometrial cell which expresses CNGB3, compared to the peptide composition including a fusion peptide between a Z13 peptide and an endosome escape peptide and a fusion peptide between a Z13 peptide and an apoptosis-inducing peptide. In addition, the present inventors found that CNGB3 is expressed not only in the endometrium and retina but also in some cancer cells.

That is, the present invention provides the following cytocidal agent.
[1] A cytocidal agent comprising
   a peptide consisting of an amino acid sequence represented by SEQ ID NO: 1 and a peptide consisting of an amino acid sequence represented by SEQ ID NO: 2, the peptide consisting of an amino acid sequence represented by SEQ ID NO: 2 being directly linked to the C-terminal side of the peptide consisting of an amino acid sequence represented by SEQ ID NO: 1; or a peptide consisting of an amino acid sequence represented by SEQ ID NO: 7 and a peptide consisting of an amino acid sequence represented by SEQ ID NO: 1, the peptide consisting of an amino acid sequence represented by SEQ ID NO: 1 being linked via RR to the C-terminal side of the peptide consisting of an amino acid sequence represented by SEQ ID NO: 7, wherein the peptide consisting of an amino acid sequence represented by SEQ ID NO: 1 is an effector peptide in which a peptide having apoptosis-inducing activity and a peptide having endosomal escape activity are linked in tandem, and
   the peptide having apoptosis-inducing activity consists of the amino acid sequence from the first to the 14th amino acids in the amino acid sequence of SEQ ID NO: 1, and the peptide having endosomal escape activity consists of the amino acid sequence from the 15th to the 19th amino acids in the amino acid sequence of SEQ ID NO: 1.
[2] The cytocidal agent according to [1], in which the peptide consisting of an amino acid sequence represented by SEQ ID NO: 1 is a peptide exclusively consisting of L-amino acids, a peptide in which, in the amino acid sequence represented by SEQ ID NO: 1, the first to the 14^{th} amino acids are D-amino acids, and the 15^{th} to 19^{th} amino acids are L-amino acids, a peptide in which, in the amino acid sequence represented by SEQ ID NO: 1, the first to 14^{th} amino acids are L-amino acids, and the 15^{th} to the 19^{th} amino acids are D-amino acids, or a peptide exclusively consisting of D-amino acids.
[3] The cytocidal agent according to [1] or [2], for use in the treatment of a disease resulting from abnormal proliferation of a cell, wherein the disease is endometriosis or cancer.

### Advantageous Effects of Invention

The cytocidal agent according to the present invention can very efficiently induce apoptosis in a target cell. For this reason, the cytocidal agent is particularly effective as a therapeutic agent of a disease resulting from abnormal proliferation of a cell of endometriosis or cancer. In addition, the endometriosis model animal according to the present disclosure is useful for drug efficacy tests on therapeutic agent candidate substances of endometriosis. In addition, by the disease onset evaluation method and the biomarker according to the present disclosure, it is possible to conveniently and efficiently evaluate a disease onset possibility of a disease that specifically expresses CNGB3 such as endometriosis and cancer.

### BRIEF DESCRIPTION OF DRAWINGS

FIG. 1 is a view illustrating a measurement result of emission intensity (RLU) of a reaction solution treated with each peptide in Reference Example 1.
FIG. 2 is a view illustrating a measurement result of emission intensity (RLU) of a reaction solution treated with each peptide in Example 1.
FIG. 3 is a view illustrating a measurement result of emission intensity (RLU) of a reaction solution treated with each peptide of a peptide 8 and peptides A1 to A6 in Example 2. FIG. 4 is a view illustrating a measurement result of emission intensity (RLU) of a reaction solution treated with each peptide of the peptide 8 and peptides B1 to B7 in Example 2.
FIG. 5 is a view illustrating a measurement result of emission intensity (RLU) of a reaction solution treated with each peptide in Example 3.
FIG. 6 is a view illustrating a measurement result of the number of alive cells of A431-CNGB3-myc cells treated with a peptide A2 in Example 4.
FIG. 7 is a staining image in which Apop-Tag assay and nuclear staining were performed on the A431-CNGB3-myc cells treated with the peptide A2 in Example 4.
FIG. 8 is a view illustrating a measurement result of emission intensity (RLU) of peritoneum of an endometriosis model mouse in which the peptide A2 was administered in the peritoneal cavity one time in Example 5.
FIG. 9 is a view illustrating a measurement result of emission intensity (RLU) of peritoneum of an endometriosis model mouse in which the peptide A2 was administered in the peritoneal cavity multiple times in Example 5.
FIG. 10 is a view illustrating a measurement result of a relative ATP amount (%) of the A431-CNGB3-myc cells treated with each peptide in Example 6.
FIG. 11 is a view illustrating a measurement result of a relative ATP amount (%) of Ishikawa cells treated with each peptide in Example 6.
FIG. 12 is a view illustrating a measurement result of a relative ATP amount (%) of SNG-II cells treated with each peptide in Example 6.
FIG. 13 is a view illustrating a measurement result of a relative ATP amount (%) of Hec-1A cells treated with each peptide in Example 6.
FIG. 14 is a view illustrating a measurement result of a relative ATP amount (%) of RL95-2 cells treated with each peptide in Example 6.
FIG. 15 is a view illustrating a result of time-dependently measuring an increase rate (%) of the number of photons of a cancer-carrying mouse to which an IF7 (RR)-(K+H) peptide is administered in Example 8.
FIG. 16 is a view illustrating a result of time-dependently measuring an increase rate (%) of a tumor volume of the cancer-carrying mouse to which the IF7 (RR)-(K+H) peptide is administered in Example 8.

### BEST MODE FOR CARRYING OUT THE INVENTION

### <Cytocidal agent>

The cytocidal agent according to the present disclosure has a peptide consisting of an amino acid sequence (KLAKLAKKLAKLAKHLAHL) represented by SEQ ID NO: 1 (hereinafter, referred to as "effector peptide") and a site selectively binding to a target molecule. The effector peptide is a peptide in which a peptide having an apoptosis-inducing activity and a peptide having an endosome escape activity are linked in tandem. The cytocidal agent according to the present disclosure binds to a target molecule through a site selectively binding to the target molecule and is taken into the target cell by endocytosis. Subsequently, as a result of destructing an endosome membrane by the action of the peptide site having endosome escape activity, the cytocidal agent included in endosome taken into the target cell is released in cytoplasm of the target cell. The cytocidal agent released in cytoplasm induces apoptosis of the target cell by interfering with mitochondrial membrane by the action of the peptide site having apoptosis-inducing activity.

The cytocidal agent according to the present disclosure includes all of a peptide moiety having apoptosis-inducing activity, a peptide moiety having endosome escape activity, and a site selectively binding to a target molecule in a molecule. For this reason, it is possible to very efficiently induce apoptosis in a target cell, compared to a peptide composition each independently including a peptide in which a peptide selectively binding to a target molecule is linked to a peptide having apoptosis-inducing activity and a peptide in which a peptide selectively binding to a target molecule is linked to a peptide having endosome escape activity.

In the present invention and the present specification, the "target cell" is a subject cell in which apoptosis is induced. The "target molecule" is a molecule present on a surface of a subject cell in which apoptosis is induced or a tissue surface on which the cell is present, and is a molecule to which the cytocidal agent according to the present invention selectively binds.

As disclosed in Patent Literature 1, a peptide (hereinafter, referred to as "KLAK peptide") consisting of an amino acid sequence (hereinafter, referred to as "KLAK sequence") consisting of 4 amino acid repeats of KLAK has an action (apoptosis-inducing activity) of inducing apoptosis by interfering with mitochondrial membrane. In addition, a peptide (hereinafter, referred to as "HLAH peptide") consisting of an amino acid sequence (hereinafter, referred to as "HLAH sequence") consisting of 4 amino acid repeats of HLAH has an action (endosome escape activity) of destructing the endosome membrane. The peptide in which the KLAK peptide is linked to the HLAH peptide has endosome escape activity and apoptosis-inducing activity. However, the intensity of the respective activities is affected by the length of amino acids in each peptide and the order of the linkage. Both of the peptide in which the HLAH peptide is linked to a downstream (C-terminal) of the KLAK peptide and the peptide in which the KLAK peptide is linked to a downstream (C-terminal) of the HLAH peptide has endosome escape activity and apoptosis-inducing activity, but the peptide in which the HLAH peptide is linked to a downstream (C-terminal) of the KLAK peptide has high apoptosis-inducing activity.

The effector peptide consisting of an amino acid sequence represented by SEQ ID NO: 1 is a peptide in which an HLAH sequence consisting of 5 amino acids is linked to a downstream of the KLAK sequence consisting of 14 amino acids. That is, in the amino acid sequence of SEQ ID NO: 1, the first to the 14^{th} amino acids are sites having apoptosis-inducing activity and the 15^{th} to the 19^{th} amino acids are sites having endosome escape activity. In order to obtain the highest apoptosis-inducing activity in a case of being taken into the target cell, the effector peptide is a peptide in which the length of the KLAK sequence, the length of the HLAH sequence, and the order of linkage of the KLAK sequence and the HLAH sequence are optimized. Since the cytocidal agent has the effector peptide, the cytocidal agent according to the present invention has very high apoptosis-inducing activity.

The effector peptide included in the cytocidal agent according to the present disclosure is not particularly limited as long the peptide is a peptide consisting of an amino acid sequence represented by SEQ ID NO: 1, may be a peptide consisting of a L-amino acid, may be a peptide consisting of a D-amino acid, or may be a peptide consisting of a L-amino acid and a D-amino acid. Since stability is high in the endosome and higher apoptosis-inducing activity is obtained, the effector peptide is preferably a peptide consisting of at least some D-amino acids, at least one of the KLAK sequence (in the amino acid sequence represented by SEQ ID NO: 1, the first to the 14^{th} amino acids) and the HLAH sequence (in the amino acid sequence represented by SEQ ID NO: 1, the 15^{th} to the 19^{th} amino acids) is more preferably a peptide consisting of a D-amino acid, and all thereof is particularly preferably a peptide consisting of a D-amino acid.

In the cytocidal agent according to the present disclosure, the effector peptide including a site selectively binding to a target molecule and an amino acid sequence represented by SEQ ID NO: 1 may directly bind, or may indirectly bind via a linker. The linker is not particularly limited, and examples thereof include a peptide of 1 to 20 amino acids, a sugar chain, polyethylene glycol, polyolefin, and the like. Since synthesis is relatively easy, as the cytocidal agent according to the present disclosure, those in which a site selectively binding to a target molecule is a peptide or protein, and which binds to the effector peptide directly or via a peptide of 1 to 20 amino acids are preferable. In addition, in the cytocidal agent according to the present disclosure, the site selectively binding to a target molecule may be linked to an N-terminal side of the effector peptide consisting of an amino acid sequence represented by SEQ ID NO: 1, or may be linked to a C-terminal side as long as binding properties to the target molecule is not hindered.

The site selectively binding to a target molecule in the cytocidal agent according to the present disclosure is determined according to the target molecule.

The target molecule to which the cytocidal agent according to the present disclosure selectively binds is a molecule present on a surface of a target cell or tissue in which apoptosis is induced.

Selectivity of the cytocidal agent according to the present disclosure becomes high, and thus side effects are decreased in a case of being used as a therapeutic agent of various diseases. From this, as the target molecule to which the cytocidal agent according to the present disclosure selectively binds, a molecule, in which an expression amount on a surface of a target cell or a tissue surface including the target cell is prominently large compared to the expression amount in many other cells or tissues, is preferable, and a molecule, which is specifically expressed on the surface of the target cell or the tissue surface including the target cell, is more preferable.

For example, CNGB3 is a membrane protein highly expressed only in the endometrium and retina in normal tissues. Local existence of CNGB3 in normal tissues is very biased, and it is only shown that moderate expression is confirmed in pineal bodies and weak expression is confirmed in bone marrow, choroid plexus, oviduct, eyes, ovaries, and testes. For this reason, the cytocidal agent having CNGB3 as a target molecule in the cytocidal agent according to the present disclosure is a cytocidal agent capable of specifically inducing apoptosis in the endometrium or retina, and is useful as a therapeutic agent for diseases of the endometrium or retina. In particular, since apoptosis is not induced in normal cells in the peritoneal cavity or pelvis, and apoptosis can be selectively induced in endometrial cells, it is very appropriate as a therapeutic agent for endometriosis.

In addition, CNGB3 is relatively strongly expressed in various cancer cells as well. For this reason, the cytocidal agent having CNGB3 as a target molecule in the cytocidal agent according to the present disclosure has high selectivity with respect to a cancer cell that expresses CNGB3 and is useful as an anti-cancer drug in which side effects are suppressed. Examples of cancers that expresses CNGB3 include uterine cancer, cervical cancer, pelvic cavity cancer, ovarian cancer, breast cancer, peritoneal wall cancer, omentum majus tumor, esophagus cancer, stomach cancer, small intestinal cancer, colon cancer, rectal cancer, appendix cancer, gall bladder cancer, pancreatic cancer, liver cancer, splenic cancer, kidney cancer, tongue cancer, pharynx cancer, nasal cancer, parotid gland cancer, thyroid cancer, malignant lymphoma, bone tumor, skin cancer, lung cancer, mediastinal cancer, testis cancer, prostate cancer, bladder cancer, brain tumor, and the like. Even cancer cells derived from the same kinds of tissues include both cancer cells which express CNGB3 and cancer cells which do not express CNGB3. For this reason, in a case where the cytocidal agent having CNGB3 as a target molecule in the cytocidal agent according to the present disclosure is used as an anti-cancer drug, it is preferable to check in advance by biopsy and the like that a target cancer cell is a cell which expresses CNGB3.

In a case where CNGB3 is used as a target molecule, examples of the site selectively binding to the target molecule include a peptide consisting of an amino acid sequence represented by SEQ ID NO: 2 (VRRAXNXPG; X represents an optional amino acid present naturally), a peptide consisting of an amino acid sequence obtained by partially modifying the amino acid sequence represented by SEQ ID NO: 2 (hereinafter, the peptide is referred to as "CNGB3-binding peptide"), and the like (Patent Literature 1). Examples of the peptide consisting of an amino acid sequence obtained by partially modifying the amino acid sequence represented by SEQ ID NO: 2 include a peptide in which 1, 2, or 3 amino acids of the amino acid sequence represented by SEQ ID NO: 2 are deleted, replaced, or added, and maintains binding properties to CNGB3; a peptide which has at least 75% or more, preferably 85% or more, and more preferably 90% or more sequence identity to the amino acid sequence represented by SEQ ID NO: 2 and maintains binding properties to CNGB3; and the like. Specific examples of the CNGB3-binding peptide include a peptide consisting of an amino acid sequence represented by SEQ ID NO: 3 (VRRADNRPG) (hereinafter, referred to as "Z13 peptide"), a peptide consisting of an amino acid sequence represented by SEQ ID NO: 4 (VRRAENRPG), a peptide consisting of an amino acid sequence represented by SEQ ID NO: 5 (VRRANNLPG), a peptide consisting of an amino acid sequence represented by SEQ ID NO: 6 (VRRANNRPG), and the like.

From a viewpoint of stronger apoptosis-inducing activity in a cell which expresses CNGB3, as the cytocidal agent having CNGB3 as a target molecule, a peptide in which the Z13 peptide and the effector peptide are linked directly or indirectly via a linker is preferable, a peptide in which the Z13 peptide and the effector peptide are linked directly or via a peptide consisting of 1 to 20 amino acids is more preferable, a peptide in which the Z13 peptide is linked to the C-terminal of the effector peptide directly or via 1 to 20 amino acids are further more preferable, a peptide in which the C-terminal of the effector peptide is directly linked to the N-terminal of the Z13 peptide (SEQ ID NO: 26: KLAKLAKKLAKLAKHLAHLVRRADNRPG) is even further more preferable, and a peptide in which the C-terminal of the effector peptide exclusively consisting of D-amino acids is directly linked to the N-terminal of the Z13 peptide exclusively consisting of all L-amino acids is particularly preferable.

In addition, annexin I is a membrane protein which is expressed in cytoplasm in normal vascular endothelial cells, but is specifically expressed on the blood stream side of the vascular endothelial cells of new blood vessels (new tumor blood vessel) in malignant tumor tissues. A molecule selectively binding to the annexin I administered into a living body is taken into the vascular endothelial cells of new tumor blood vessels via a bond to annexin I expressed on the blood stream side of the new tumor blood vessels in the malignant tumor tissues. The molecule taken into the vascular endothelial cells are transported from the apical side to the basal side by vesicle transport and discharged to interstitial cells. The discharged molecule is diffused in interstitial cells and taken into tumor cells. For this reason, a cytocidal agent having annexin I as a target molecule in the cytocidal agent according to the present disclosure is a cytocidal agent capable of inducing apoptosis in a cancer cell, and is useful as an anti-cancer drug. The cytocidal agent having annexin I as a target molecule is specifically taken into the vascular endothelial cells of new tumor blood vessels, and in the vesicle transport process in the vascular endothelial cells or in the vesicle transport process in the taken tumor cells, a membrane of endosome is destructed by a function of HLAH peptide moiety and released into cytoplasm. After that, by a function of KLAK peptide moiety, cells into which the cytocidal agent is taken are killed. In a case where a cytocidal agent having annexin I as a target molecule is used as an anti-cancer drug, cancers which are therapeutic subjects are not particularly limited, and can be used for the same cancer as those exemplified described above.

In a case of having annexin I as a target molecule, examples of a site selectively binding to a target molecule include a peptide consisting of an amino acid sequence represented by SEQ ID NO: 7 (IFLLWQR) (hereinafter, referred to as "IF7 peptide") and a peptide consisting of an amino acid sequence in which a moiety of the amino acid sequence represented by SEQ ID NO: 7 is modified (hereinafter, the peptide is referred to as "annexin I-binding peptide"). Examples of the peptide consisting of an amino acid sequence in which a moiety of the amino acid sequence represented by SEQ ID NO: 7 is modified include a peptide in which one, two, or three amino acids of the amino acid sequence represented by SEQ ID NO: 7 are deleted, substituted, or added and which maintains binding performance to annexin I or a peptide which has at least 70% or more, and preferably 85% or more sequence identity with the amino acid sequence represented by SEQ ID NO: 7 and maintains binding performance to annexin I.

From a viewpoint of having stronger apoptosis-inducing activity to cancer cells, the cytocidal agent having annexin I as a target molecule is preferably a cytocidal agent in which an IF7 peptide and the effector peptide are linked directly or indirectly via a linker, more preferably a peptide in which the IF7 peptide and the effector peptide are linked directly or via a peptide consisting of 1 to 20 amino acids, even more preferably a peptide in which the effector peptide is linked to a C-terminal of the IF7 peptide directly or via a peptide consisting of 1 to 20 amino acids, still even more preferably a peptide in which the effector peptide is linked to a C-terminal of the IF7 peptide via a peptide consisting of 1 to 5 amino acids, and particularly preferably a peptide in which a C-terminal of the IF7 peptide exclusively consisting of L-amino acids is linked to a N-terminal of the effector peptide exclusively consisting of D-amino acids via a peptide consisting of 1 to 5 L-amino acids. Examples of the peptide in which the C-terminal of the IF7 peptide is linked to the N-terminal of the effector peptide via a peptide consisting of 1 to 5 amino acids include an amino acid sequence (IFLLWQRRRKLAKLAKKLAKLAKHLAHL) represented by SEQ ID NO: 40.

In a case where the cytocidal agent according to the present disclosure is for use as a therapeutic drug, the administration path is not particularly limited, and appropriately determined depending on the target cell and tissues including thereof. Examples of the administration path of the cytocidal agent according to the present disclosure include oral administration, intravenous administration, intraperitoneal administration, enema administration, and the like.

The cytocidal agent according to the present disclosure can be formulated as an oral solid agent such as a powder, a granule, a capsule, a tablet, and a chewable agent, an oral liquid agent such as a solution agent and a syrup agent, an injection, an enema agent, a spray agent, a patch, and an ointment by a general method.

The cytocidal agent according to the present disclosure is formulated by being mixed with an excipient, a binding agent, a lubricant, a disintegrating agent, a fluidizing agent, a solvent, a solubilizing agent, a buffer, a suspending agent, an emulsifier, an isotonizing agent, a stabilizer, an antiseptic agent, an anti-oxidant, a flavoring agent, a coloring agent, and the like, depending on the formulation necessity.

Examples of the excipient include saccharides such as lactose, glucose, and D-mannitol, celluloses such as starch and crystalline cellulose, sugar alcohols such as erythritol, sorbitol, and xylitol, dicalcium phosphate, calcium carbonate, and kaoline. Examples of the binding agent include pregelatinized starch, gelatin, Arabic rubber, methyl cellulose, carboxy methyl cellulose, sodium carboxy methyl cellulose, crystalline cellulose, D-mannitol, trehalose, hydroxy propyl cellulose, hydroxy propyl methyl cellulose, polyvinyl pyrrolidone, polyvinyl alcohol, and the like. Examples of the lubricant include stearic acid, calcium stearate, talc, sucrose fatty acid ester, polyethylene glycol, and the like. Examples of the disintegrating agent include crosslinking polyvinyl pyrrolidone (crospovidone), low substitution degree hydroxy propyl cellulose, starch, alginic acid, sodium alginate, and the like. Examples of the fluidizing agent include silicic acid, silicic anhydride, aluminum silicate, calcium silicate, a magnesium metasilicate aluminate compound, an aluminum oxide, an aluminum hydroxide, a magnesium oxide, a magnesium hydroxide, and the like. Examples of the solvent include purified water, a physiological saline solution, and the like. Examples of the solubilizing agent include dextran, polyvinyl pyrrolidone, sodium benzoate, ethylene diamine, salicylate amide, nicotinic acid amide, a polyoxy ethylene hydrogenated castor oil derivative, and the like. Examples of the buffer include sodium citrate hydrate, sodium acetate hydrate, sodium hydrogen carbonate, trometamol, boric acid, borax, dibasic sodium phosphate hydrate, sodium dihydrogen phosphate, and the like. Examples of the suspending agent or the emulsifier include sodium lauryl sulfate, Arabic rubber, gelatin, lecithin, glyceryl monostearate, polyvinyl alcohol, polyvinyl pyrrolidone, celluloses such as sodium carboxy methyl cellulose, polyoxy ethylene hydrogenated castor oil, and the like. Examples of the isotonizing agent include saccharides such as lactose, glucose, and D-mannitol, sodium chloride, potassium chloride, glycerin, propylene glycol, polyethylene glycol, urea, and the like. Examples of the stabilizer include polyethylene glycol, sodium dextran sulfate, sodium sulfite, and the like. Examples of the antiseptic agent include para-hydroxybenzoic esters, chlorobutanol, benzyl alcohol, phenethyl alcohol, chlorocresol, dehydroacetic acid, sorbic acid, and the like. Examples of the anti-oxidant include sulfite, ascorbic acid, and the like. Examples of the flavoring agent include sweeteners generally used in the field of the therapeutic drug and the food, aromatic chemical, and the like. Examples of the coloring agent include coloring materials generally used in the field of the therapeutic drug and the food.

The cytocidal agent according to the present disclosure may be used as it is, and can be also used as a therapeutic composition including other components. Examples of the other components included in the therapeutic composition include the excipient, the binding agent, the lubricant, the disintegrating agent, the fluidizing agent, the solvent, the solubilizing agent, the buffer, the suspending agent, the emulsifier, the isotonizing agent, the stabilizer, the antiseptic agent, the anti-oxidant, the flavoring agent, the coloring agent, and the like. In addition, the therapeutic composition may contain other effective components than the cytocidal agent according to the present disclosure.

The cytocidal agent according to the present disclosure is preferably administered to mammals, more preferably administered to humans or livestock such as mouse, rat, rabbit, guinea pig, hamster, monkey, sheep, horse, cattle, pig, donkey, dog, and cat, and further more preferably administered to humans.

EXAMPLES Subsequently, the present invention will be further explained in detail using examples.

### <Endometriosis model cell (A431-CNGB3-myc cell)>

A transformed cell (A431-CNGB3-myc cell) obtained by introducing a gene encoding human CNGB3, in which an myc tag was fused in a C-terminal, into an A431 cell (human epithelial-like cell cancer-derived cell strain) and forcibly expressing thereof was prepared as an endometriosis model cell.

Culturing of the A431-CNGB3-myc cell was performed at 37°C in a 5 volume% carbon dioxide environment using a medium in which 10% of inactivated FBS (bovine fetal serum, manufactured by Corning Corporation) and 1% of penicillin-streptomycin (manufactured by Invitrogen Corporation) were contained in a DMEM High Glucose medium (manufactured by GIBCO Corporation), as a culture medium. Passage was performed every two or three days.

### <Endometriosis model mouse>

A431-CNGB3-myc cells were transplanted to a peritoneal cavity of an immunodeficient mouse (NOD/ShiJic-scid Jcl strain, supplied by CLEA Japan, Inc.) to prepare an endometriosis model mouse.

Specifically, after thawing frozen-stored A431-CNGB3-myc cells, a cell solution prepared by adding a culture medium to cells passaged twice using a 10-cm dish (manufactured by Thermo Fisher Scientific Co., Ltd., Lot No. F3BAXQ103) so as to be 1 × 10⁷ cells/0.5 mL/body was used as an administration solution. The administration solution was intraperitoneally administered to a 7-week-old female immunodeficient mouse as soon as possible after preparation to transplant the A431-CNGB3-myc cells.

For the mouse, 5 to 10 mice/cage in a polycarbonate cage (W × D × H = 270 × 440 × 187 (mm)) were bred in an environment of 19.8°C to 27.1°C, humidity of 32% to 75%, and 12 hours of light. Feed (sterilized CRF-1 (solid type), manufactured by Oriental Yeast Co., Ltd.) and drinking water (sterile tap water) were freely ingested.

Model preparation was confirmed by observation and collection of a peritoneal tumor. One to three weeks after A431-CNGB3-myc cell transplantation, autopsy was performed on each two mice a week to visually check whether a tumor (granular shape of about 1 mm) was observed in the peritoneum and photographed. Thereafter, the peritoneum was collected, and 4 locations (each on the left and right sides of the abdomen and back) were cut out, immersed and fixed in 10% neutral buffer formalin, respectively, and refrigerated. The peritoneum after formalin fixation was subjected to immunohistochemical staining using an anti-c-myc antibody to examine the status of dissemination.

As a result of visual checking of the mouse after A431-CNGB3-myc cell transplantation, in the first test, although a tumor was visually confirmed 1 week after transplantation, no peritoneal dissemination was observed, but two or three weeks after transplantation, a tumor and peritoneal dissemination were visually confirmed. In the second test, peritoneal dissemination was visually confirmed even one week after transplantation. In addition, as a result of c-myc staining of the peritoneal tissue fragment of the mouse after transplantation, it was confirmed that one week after the transplantation, peritoneal dissemination was already proceeded.

### [Reference Example 1]

In a peptide in which a KLAK peptide, an HLAH peptide, and a CNGB3-binding peptide were linked, the impact of the type of the amino acid sequence of the CNGB3-binding peptide on the intensity of the cytotoxicity against endometriosis cells was examined.

Specifically, two types of peptides consisting of the amino acid sequences shown in Table 2 were synthesized, and cytotoxicity against A431-CNGB3-myc cells was compared. In the two types of peptides, the KLAK sequence and the HLAH sequence moiety were synthesized from D-amino acids, and the CNGB3-binding peptide moiety was synthesized with L-amino acids.

**[Table 1]**

| | KLAK-HLAH | Seq. | Seq. No. |
|---|---|---|---|
| WT | 14+14 | KLAKLAK-KLAKLAK-HLAHLAH-HLAHLAH-VRRADNRPG | 8 |
| D5E | 14+14 | KLAKLAK-KLAKLAK-HLAHLAH-HLAHLAH-VRRAENRPG | 9 |

### <Evaluation of cytocidal properties>

The cytocidal activity of each peptide was evaluated by measuring an ATP amount in A431-CNGB3-myc cells treated with each peptide using CellTiter-Glo (registered trademark) assay kit (manufactured by Promega).

Specifically, first, A431-CNGB3-myc cells after 2 passages were disseminated into a 96-well plate (manufactured by Corning/Costar, Lot No. 00515003) so as to be 1 × 10⁴ cells/well by preparing the concentration of the cells. After culturing for 2 days after the dissemination, each peptide was added to each well such that a final concentration was 35.5, 75, or 150 µM, respectively, and cultured for 24 hours. Thereafter, the culture supernatant was removed from each well, and CellTiter-Glo buffer was added to the remaining cells, and after homogenization, the supernatant was recovered by centrifugation and used as a lysate. A double quantity of PBS and a 2 × CellTiterGlo Reagent of the equivalent amount of the lysate were added to the lysate and stirred to obtain a reaction solution, and the reaction solution was stood at room temperature for 10 minutes. The luminescence intensity (Luminescence) (RLU: RELATIVE LIGHT UNITS) of the reaction solution after standing at room temperature was measured using a Synergy H1 hybrid multi-mode microplate reader (manufactured by BioTek Corporation). The luminescence intensity of the reaction solution is an indicator of the amount of ATP. It is shown that the smaller the luminescence intensity of the reaction solution is, the amount of ATP is small, and the cytocidal activity of the administered peptide is strong. All trials were measured in triplicate and the average was evaluated as the cytocidal activity at the concentration of each peptide.

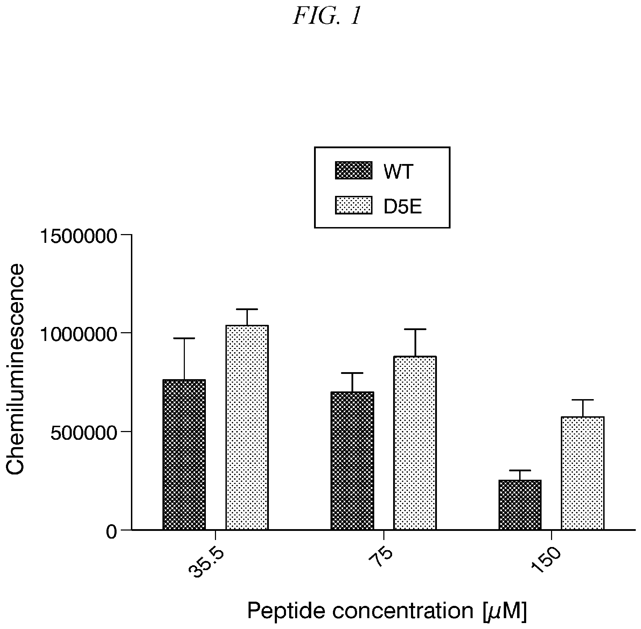

In the evaluation of the cytocidal activity against the A431-CNGB3-myc cells of each peptide, the measurement result of the fluorescence intensity (RLU) of each reaction solution is shown in FIG. 1. In FIG. 1, the "WT" indicates the result of the peptide WT of Table 1, and the "DSE" indicates the result of the peptide D5E of Table 1, respectively. Both peptides commonly showed concentration-dependent cytocidal activity. The peptide WT in which the CNGB3-binding peptide is a Z13 peptide showed stronger cytocidal activity than the peptide D5E in which the CNGB3-binding peptide is a peptide of SEQ ID NO: 4.

### [Example 1]

In the peptide in which the KLAK peptide, the HLAH peptide, and the Z13 peptide were linked, a length of the KLAK sequence and the HLAH sequence was optimized such that the intensity of cytotoxicity against the endometriosis cells became the maximum.

Specifically, 16 types of peptides consisting of an amino acid sequence shown in Table 2 was synthesized, and cytotoxicity against the A431-CNGB3-myc cells was compared. In the 16 types of peptides, the KLAK sequence and the HLAH sequence moiety were synthesized from D-amino acids, and the Z13 peptide moiety was synthesized with L-amino acids.

**[Table 2]**

| Peptide | KLAK-HLAH | Seq. | Seq. No. |
|---|---|---|---|
| 1 | 14+14 | KLAKLAK-KLAKLAK-HLAHLAH-HLAHLAH-VRRADNRPG | 8 |
| 2 | 14+13 | KLAKLAK-KLAKLAK-HLAHLAH-HLAHLA-VRRADNRPG | 10 |
| 3 | 14+12 | KLAKLAK-KLAKLAK-HLAHLAH-HLAHL-VRRADNRPG | 11 |
| 4 | 14+11 | KLAKLAK-KLAKLAK-HLAHLAH-HLAH-VRRADNRPG | 12 |
| 5 | 14+10 | KLAKLAK-KLAKLAK-HLAHLAH-HLA-VRRADNRPG | 13 |
| 6 | 14+9 | KLAKLAK-KLAKLAK-HLAHLAH-HL-VRRADNRPG | 14 |
| 7 | 14+8 | KLAKLAK-KLAKLAK-HLAHLAH-H-VRRADNRPG | 15 |
| 8 | 14+7 | KLAKLAK-KLAKLAK-HLAHLAH-VRRADNRPG | 16 |
| | HLAH-KLAK | | |
| 9 | 14+14 | HLAHLAH-HLAHLAH-KLAKLAK-KLAKLAK-VRRADNRPG | 17 |
| 10 | 14+13 | HLAHLAH-HLAHLAH-KLAKLAK-KLAKLA-VRRADNRPG | 18 |
| 11 | 14+12 | HLAHLAH-HLAHLAH-KLAKLAK-KLAKL-VRRADNRPG | 19 |
| 12 | 14+11 | HLAHLAH-HLAHLAH-KLAKLAK-KLAK-VRRADNRPG | 20 |
| 13 | 14+10 | HLAHLAH-HLAHLAH-KLAKLAK-KLA-VRRADNRPG | 21 |
| 14 | 14+9 | HLAHLAH-HLAHLAH-KLAKLAK-KL-VRRADNRPG | 22 |
| 15 | 14+8 | HLAHLAH-HLAHLAH-KLAKLAK-K-VRRADNRPG | 23 |
| 16 | 14+7 | HLAHLAH-HLAHLAH-KLAKLAK-VRRADNRPG | 24 |

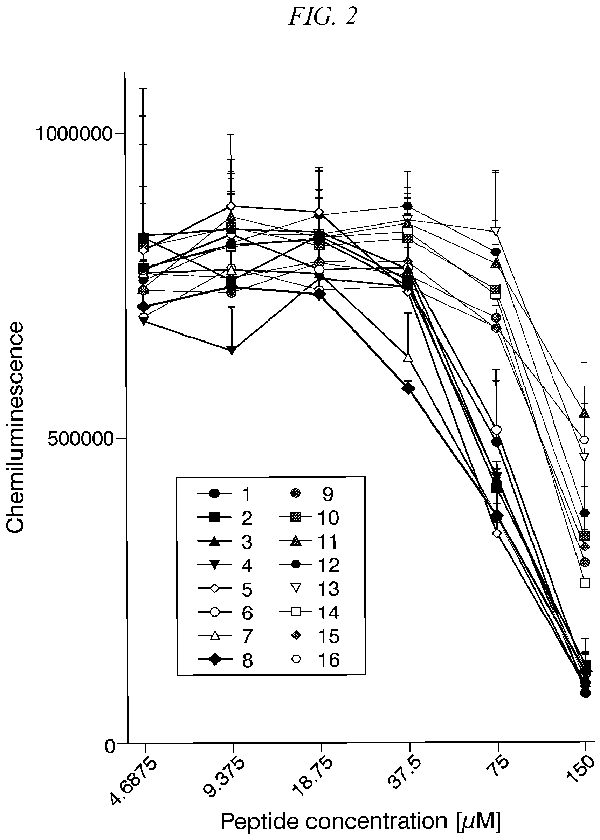

The final concentration of each peptide added to the A43 1-CNGB3-myc cells disseminated into the 96-well plate was set to be 2.34, 4.69, 9.38, 18.8, 37.5, 75, or 150 µM, and the cytocidal activity of each peptide was evaluated in the same manner as that of Reference Example 1, except that the culture time after addition of the peptide was set to 20 hours. In the evaluation of the cytocidal activity against the A431-CNGB3-myc cells of each peptide, the measurement result of the fluorescence intensity (RLU) of each reaction solution is shown in FIG. 2. In the figure, "1" to "16" show the result of the reaction solution to which peptides 1 to 16 of Table 2 were added, respectively. All of them showed concentration-dependent cytocidal activity 20 hours after the addition of the peptide. The peptides 1 to 8 in which the peptide of the HLAH sequence is linked to a downstream of the peptide of the KLAK sequence showed stronger cytocidal activity than the peptides 9 to 16 in which the peptide of the KLAK sequence was linked to a downstream of the peptide of the HLAH sequence. In addition, in the peptides 1 to 8, the shortest peptide 8 had stronger cytocidal activity than the longest peptide 1, and in the peptides 9 to 16, the shortest peptide 16 had stronger cytocidal activity than the longest peptide 9.

### [Example 2]

The peptide 8 showing the strongest cytocidal activity in Example 1 was further shortened to optimize the intensity of the cytotoxicity against the endometriosis cells to the maximum.

Specifically, 14 types of peptides consisting of the amino acid sequences shown in Table 3 were synthesized, and cytotoxicity against A431-CNGB3-myc cells was compared. In the 14 types of peptides, the KLAK sequence and the HLAH sequence moiety were synthesized from D-amino acids, and the Z13 peptide moiety was synthesized with L-amino acids.

**[Table 3]**

| Peptide | KLAK-HLAH | Seq. | Seq. No. |
|---|---|---|---|
| 8 | 14+7 | KLAKLAK-KLAKLAK-HLAHLAH-VRRADNRPG | 16 |
| A1 | 14+6 | KLAKLAK-KLAKLAK-HLAHLA-VRRADNRPG | 25 |
| A2 | 14+5 | KLAKLAK-KLAKLAK-HLAHL-VRRADNRPG | 26 |
| A3 | 14+4 | KLAKLAK-KLAKLAK-HLAH-VRRADNRPG | 27 |
| A4 | 14+3 | KLAKLAK-KLAKLAK-HLA-VRRADNRPG | 28 |
| A5 | 14+2 | KLAKLAK-KLAKLAK-BL-VRRADNRPG | 29 |
| A6 | 14+1 | KLAKLAK-KLAKLAK-H-VRRADNRPG | 30 |
| B1 | 13+7 | KLAKLAK-KLAKLA-HLAHLAH-VRRADNRPG | 31 |
| B2 | 12+7 | KLAKLAK-KLAKL-HLAHLAH-VRRADNRPG | 32 |
| B3 | 11+7 | KLAKLAK-KLAK-HLAHLAH-VRRADNRPG | 33 |
| B4 | 10+7 | KLAKLAK-KLA-HLAHLAH-VRRADNRPG | 34 |
| B5 | 9+7 | KLAKLAK-KL-HLAHLAH-VRRADNRPG | 35 |
| B6 | 8+7 | KLAKLAK-K-HLAHLAH-VRRADNRPG | 36 |
| B7 | 7+7 | KLAKLAK-HLAHLAH-VRRADNRPG | 37 |

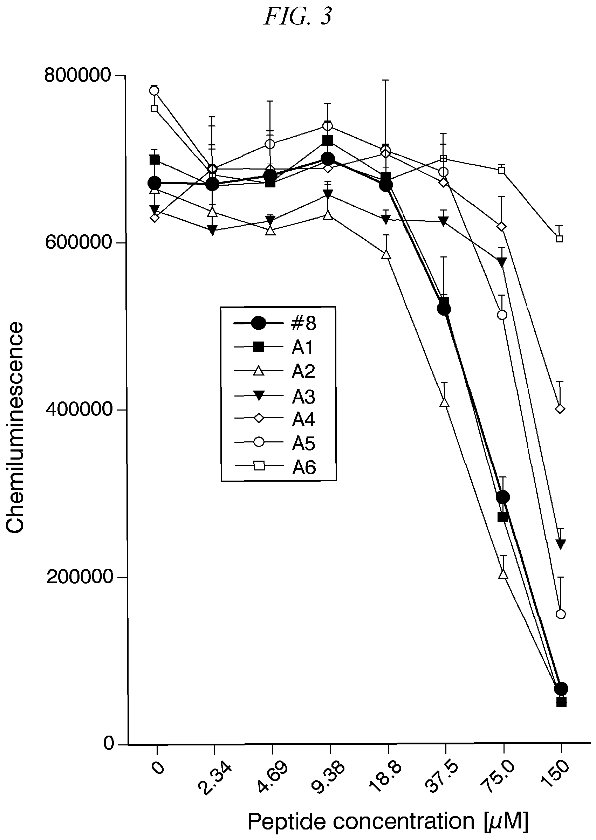

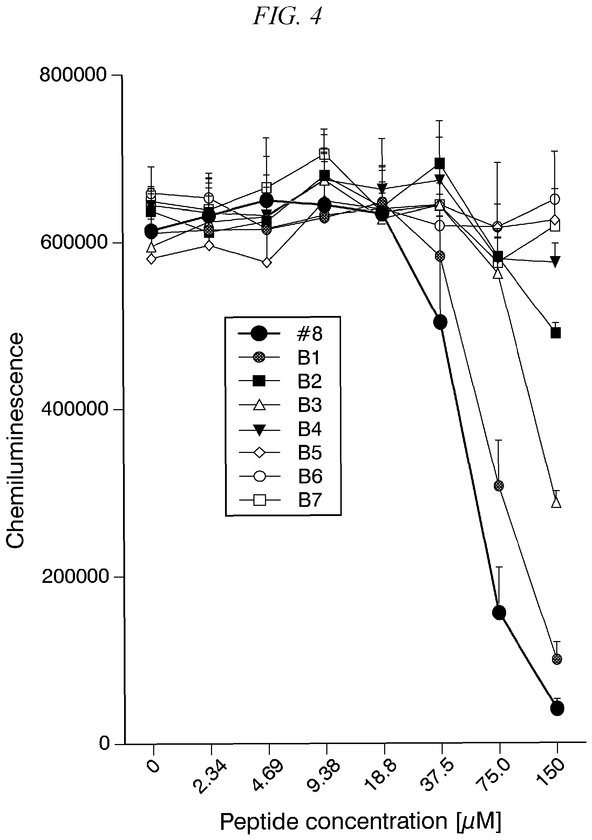

The cytocidal activity of each peptide was evaluated in the same manner as that of Example 1. In evaluation of the cytocidal activity against the A431-CNGB3-myc cells of each peptide, the measurement result of the fluorescence intensity (RLU) of the reaction solution treated with the peptide 8 and the peptides A1 to A6 was shown in FIG. 3, and the measurement result of the fluorescence intensity (RLU) of the reaction solution treated with the peptide 8 and the peptides B1 to B7 was shown in FIG. 4. In the figures, the "#8" represents the result of the reaction solution to which the peptide 8 of Table 3 was added, the "A1" to "A6" and "B1" to "B6" show the result of the reaction solution to which the peptides A1 to A6 and B1 to B6 of Table 3 were added, respectively. In the peptides A1 to A6 of which the KLAK sequence consisted of 14 amino acids, the peptides A3 to A6 of which the HLAH sequence consisted of 4 amino acids or less showed cytocidal activity even when the peptide final concentration was 75 µM (FIG. 3). In addition, in the peptides B1 to B7 of which the HLAH sequence consisted of 7 amino acids, the peptides B3 to B7 of which the KLAK sequence consisted of 11 amino acids or less showed cytocidal activity even when the peptide final concentration was 75 µM (FIG. 4). In addition, only the peptide A2 showed stronger cytocidal activity than the peptide 8. From the results, it was recognized that as the effector peptide, a peptide in which a peptide of which the KLAK sequence consists of 14 amino acids and a peptide of which the HLAH sequence consists of 5 amino acids were linked showed the highest cytocidal activity.

### [Example 3]

In the peptide in which the KLAK peptide, the HLAH peptide, and the Z13 peptide were linked, the intensity of the cytocidal activity in a case where the constituent amino acid was D-amino acid and in a case where the constituent amino acid was L-amino acid was examined.

Specifically, intensity of the cytocidal activity of the peptide A2 (both of the KLAK sequence consisting of 14 amino acids and the HLAH sequence consisting of 5 amino acids include D-amino acids. It is referred to as a peptide (14D + 5D)) described in Table 3, a peptide (14D + 5L) in which in the amino acids of the peptide 8, the KLAK sequence consisting of 14 amino acids includes D-amino acids, and the HLAH sequence consisting of 5 amino acids include L-amino acids, a peptide (14L + 5D) in which in the amino acids of the peptide 8, the KLAK sequence consisting of 14 amino acids include L-amino acids, and the HLAH sequence consisting of 5 amino acids include D-amino acids, and a peptide (14L + 5L) in which all of the amino acids of the peptide 8 include L-amino acids was examined in the same manner as that of Example 1. All of the Z13 peptide moiety of these peptides include only L-amino acids.

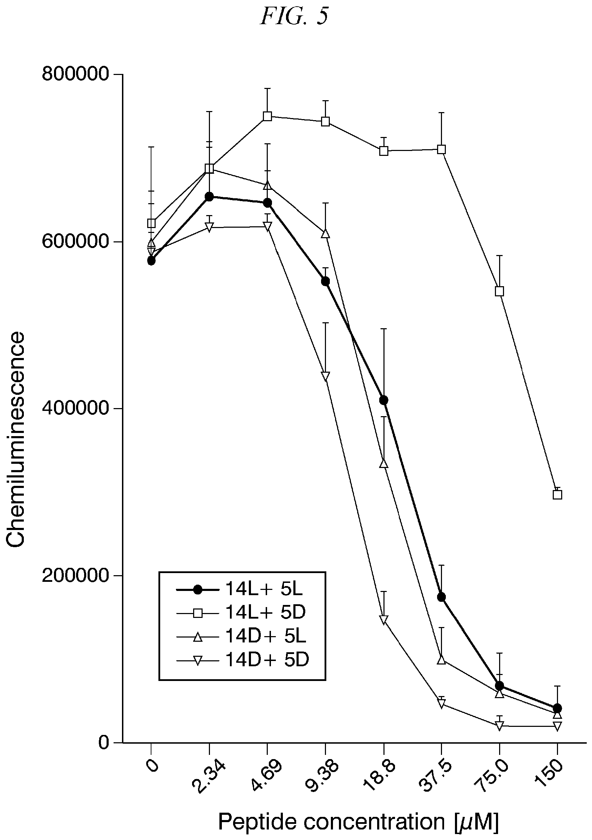

In the evaluation of the cytocidal activity against the A431-CNGB3-myc cells of each peptide, the measurement result of the fluorescence intensity (RLU) of the reaction solution treated with each peptide was shown in FIG. 5. The cytocidal activity of the peptide (14L + 5L) in which all of the escape peptides include L-amino acids was the lowest, and the cytocidal activity of the peptide A2 (peptide (14D + 5D)) in which all of the escape peptides include D-amino acids was the highest. It was estimated that the peptide consisting of D-amino acids was hardly digested in the endosome or the cytoplasm than the peptide consisting of L-amino acids, and the endosome escape activity and the apoptosis-inducing activity were sufficiently exhibited.

### [Example 4]

The cytocidal activity of the peptide A2 in which the cytocidal activity was the highest was examined in Examples 2 and 3.

### <Quantitative analysis of alive cells>

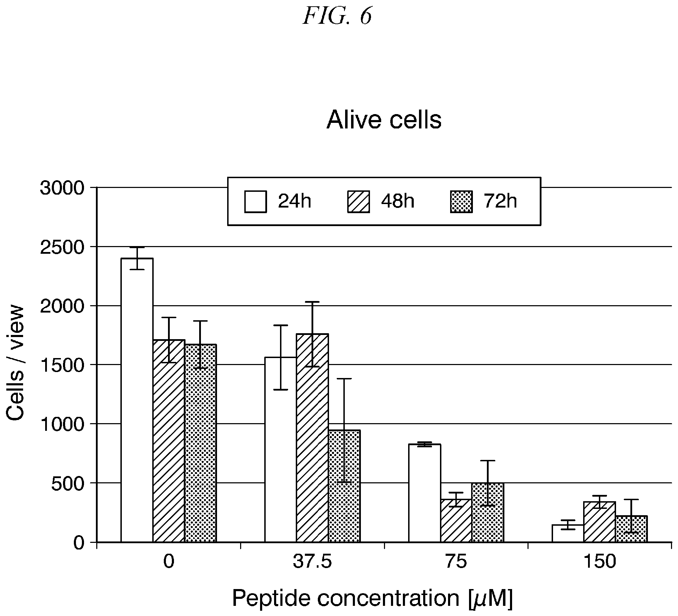

The A431-CNGB3-myc cells disseminated on a cover glass placed in a multi-dish for cell culture were added such that the final concentration of the peptide A2 was 0 (control), 37.5, 75, or 150 µM, and cultured. After 24, 48, or 72 hours from the addition of the peptide, the number of cells (alive cells) adhered and spread onto the cover glass were counted. The measurement result of the number of the alive cells (Cells/view) per single visual field is shown in FIG. 6. As a result, it was determined that the number of the alive cells of the A431-CNGB3-myc cells was decreased dependent on the concentration of the added peptide A2 over time.

### <Analysis of apoptosis-inducing activity>

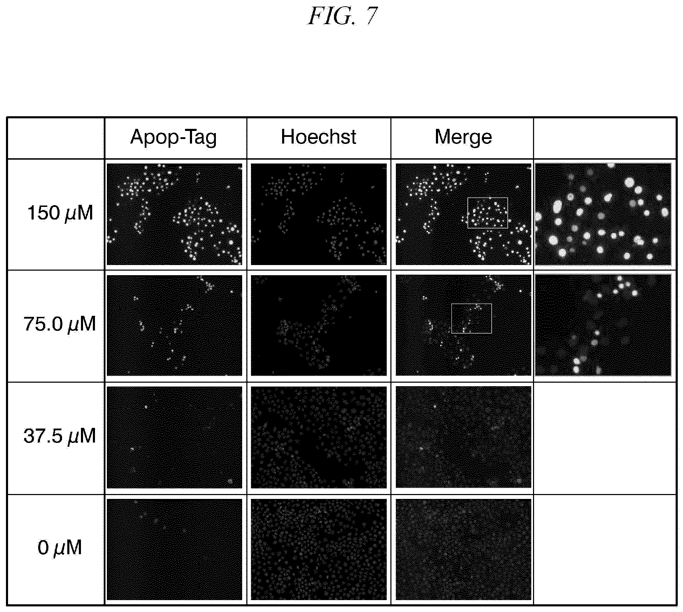

The A431-CNGB3-myc cells disseminated on a cover glass placed in a multi-dish for cell culture were added such that the final concentration of the peptide A2 was 0 (control), 37.5, 75, or 150 µM, and cultured. After 24, 48, or 72 hours from the addition of the peptide, apoptosis was detected by using ApopTag (registered trademark) Fluorescein In Situ Apoptosis Detection Kit. FIG. 7 shows the result in which Apop-Tag assay was performed on the cells after 48 hours from the addition of the peptide. In the figure, the "Hoechst" represents the result of nuclear staining with a Hoechst 33342 solution. As a result, dependent on the concentration of the added peptide A2, it was confirmed that the number of the Apop-Tag stained cells was large, and apoptosis was induced by the peptide A2.

### [Example 5]

The peptide A2 which has the highest cytocidal activity in Examples 2 and 3 was administered to the endometriosis model mouse, and a therapeutic effect on endometriosis was examined.

### <Single administration of peptide into peritoneal cavity>

0.5 mL of a physiological saline solution heated to 37°C in the peritoneum was put into the endometriosis model mouse after 7 days from transplanting the A431-CNGB3-myc cells into the peritoneal cavity under isoflurane anesthesia, and immediately after massaging, the peptide A2 was dissolved in the physiological saline solution and the solution was administered into the peritoneum. The peptide A2 was administered such that an administration amount per mouse weight was 0 mg/10 mL/kg (control), 5 mg/10 mL/kg, or 10 mg/10 mL/kg (n=3).

### <Multiple administration of peptide to peritoneal cavity>

0.5 mL of a physiological saline solution heated to 37°C in the peritoneum was put into the endometriosis model mouse after 7 days from transplanting the A431-CNGB3-myc cells into the peritoneal cavity under isoflurane anesthesia, and immediately after massaging, the peptide A2 was dissolved in the physiological saline solution and the solution was administered into the peritoneum (single administration). Thereafter, after 8 and 9 days from transplanting the A431-CNGB3-myc cells into the peritoneal cavity, the peptide A2 solution was administered in the same manner. That is, the peptide A2 solution was administered once a day, a total of three times. The peptide A2 was administered such that an administration amount per mouse weight was 0 mg/10 mL/kg (control), 2.5 mg/10 mL/kg, 5.0 mg/10 mL/kg, or 7.5 mg/10 mL/kg (n=3).

### <Collection of peritonea (material collection)>

The endometriosis model mouse into which the peptide A2 was administered died of bleeding under isoflurane anesthesia after 24 hours or 48 hours from the final administration to collect the peritoneum. The peritoneum for ATP measurement was frozen with liquid nitrogen, and stored in a deep freezer until the measurement. A peritoneum for pathological specimen preparation was immersed and fixed in 10% neutral buffer formalin, respectively, and refrigerated.

A weight of each mouse was measured before being died of bleeding. There was no particular change in the weight of each mouse regardless of the administration amount or the number of administrations of the peptide A2.

### <Measurement of ATP amount>

The measurement of the ATP amount was performed by using a CellTiter-Glo (registered trademark) assay kit (manufactured by Promega). The weight of the frozen peritoneum was measured, and 10 times the quantity of CellTiter-Glo buffer of the frozen tissue was added, and after homogenization, the supernatant was recovered by centrifugation and used as a lysate. A double quantity of PBS and a 2 × CellTiterGlo Reagent of the equivalent amount of the lysate were added to the lysate and stirred to obtain a reaction solution, and the reaction solution was stood at room temperature for 10 minutes. The luminescence intensity (RLU) of the reaction solution after standing at room temperature was measured using a Synergy H1 hybrid multi-mode microplate reader (manufactured by BioTek Corporation). It is shown that the luminescence intensity of the reaction solution is an indicator of the amount of ATP, and the smaller the luminescence intensity of the reaction solution is, the amount of ATP is small, and the cytocidal activity of the administered peptide is strong. All trials were measured in triplicate (n=3) and the average was evaluated as the cytocidal activity of each peptide.

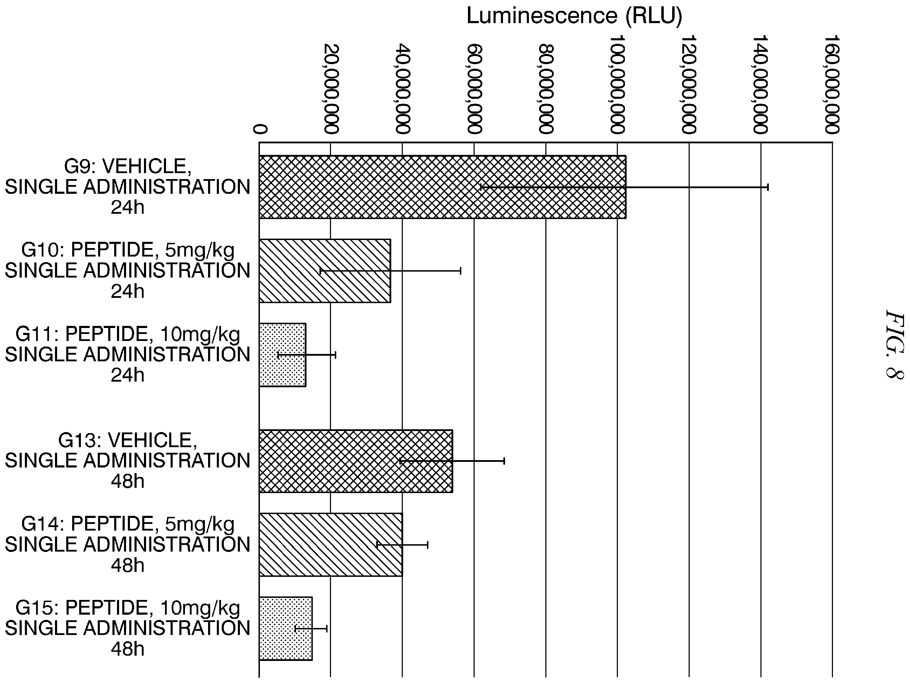

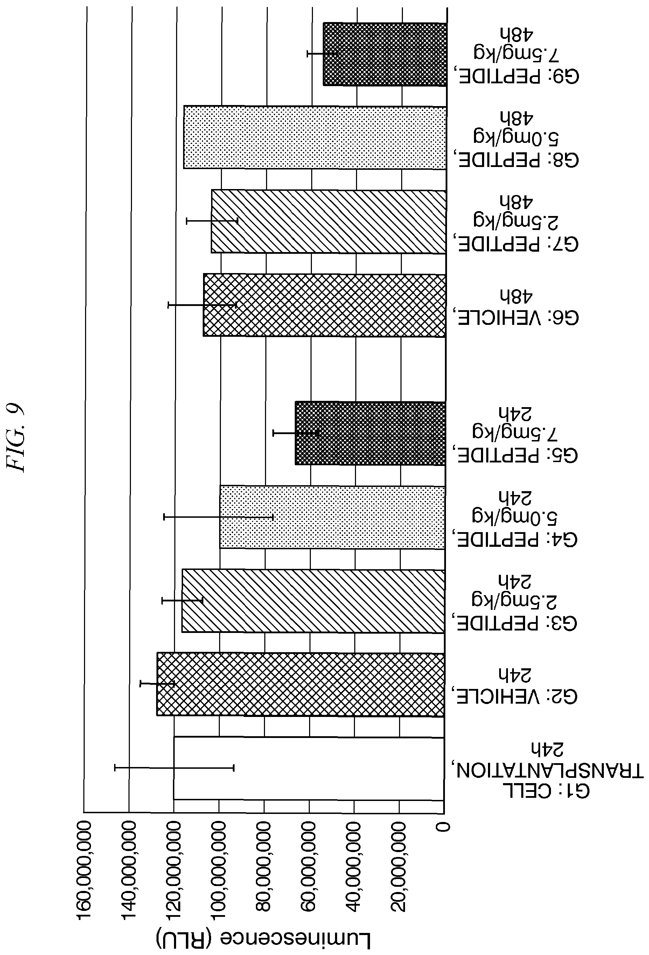

The measurement result of the luminescence intensity (RLU) of the peritoneum of the endometriosis model mouse into which the peptide A2 was administered once is shown in FIG. 8. In addition, the measurement result of the luminescence intensity (RLU) of the peritoneum of the endometriosis model mouse into which the peptide A2 was repeatedly administered three times is shown in FIG. 9. As shown in FIGS. 8 and 9, in any of a mouse to which administration was performed once and a mouse to which administration was performed multiple times, it was confirmed that the luminescence intensity (RLU) of the peritoneum was decreased dependent on the administered peptide A2 amount, and the amount of ATP in the peritoneum was decreased. The decrease in the amount of ATP in the peritoneum means reduction of the number of the alive cells of the A431-CNGB3-myc cells transplanted into the peritoneum. From the result, it can be said that the A431-CNGB3-myc cells in the peritoneal cavity was killed by the peptide A2, that is, the peptide A2 can exhibit cytocidal effect in an in-vivo environment, and is particularly useful as a therapeutic agent for endometriosis.

### <TUNEL staining>

HE staining and TUNEL staining were performed on the peritoneum for pathological specimen preparation fixed with 10% neutral buffer formalin. As a result, it was confirmed that a peptide A2 administration group is TUNEL method (TdT-mediated Dutp nick end labeling) positive (not illustrated), and the peptide A2 induces apoptosis and shows a cytocidal effect.

### [Example 6]

A cytocidal activity of the peptide A2 (peptide (14D + 5D)) to the A431-CNGB3-myc cells and a cell strain derived from various uterine cancer cells was examined.

As the cancer-derived cell strain, an Ishikawa cell derived from human endometrium gland cancer, an SNG-II cell derived from human endometrium cancer, an Hec-1A cell derived from human uterine body cancer, and a RL95-2 cell derived from human endometrium cancer were used. The cells were cultured in the same manner as the A431-CNGB3-myc cells.

As the peptide treating each cell, three types of peptides consisting of amino acid sequences shown in Table 4 were used. In the three types of peptides, the KLAK sequence and the HLAH sequence moiety were synthesized from D-amino acids, and the Z13 peptide moiety was synthesized with L-amino acids.

**[Table 4]**

| | KLAK-HLAH-Z13 | Seq. | Seq. No. |
|---|---|---|---|
| A2 | 14D+5D+Z13 | KLAKLAK-KLAKLAK-HLAHL-VRRADNRPG | 26 |
| A2-1 | 14D+Z13 | KLAKLAK-KLAKLAK- VRRADNRPG | 38 |
| A2-2 | 5D+Z13 | HLAHL-VRRADNRPG | 39 |

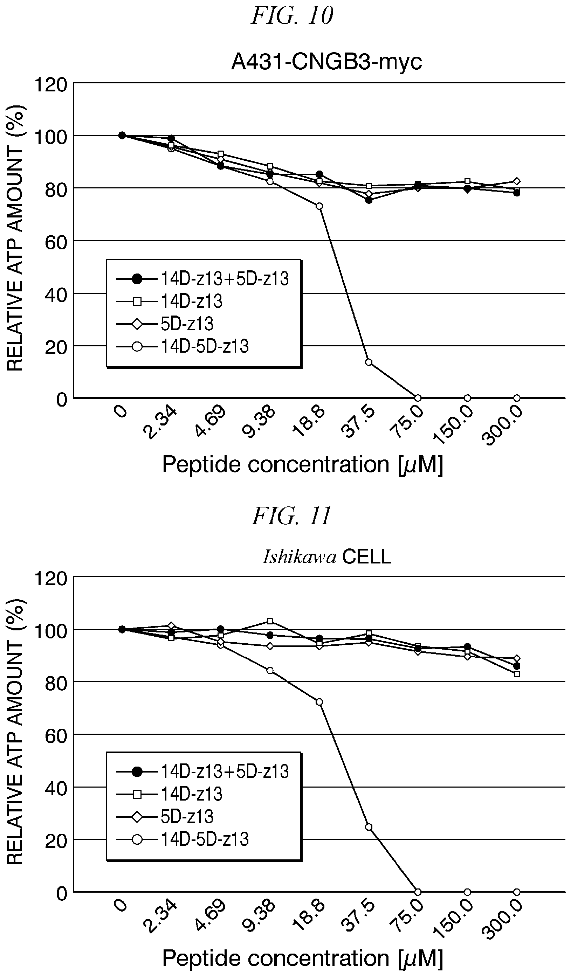

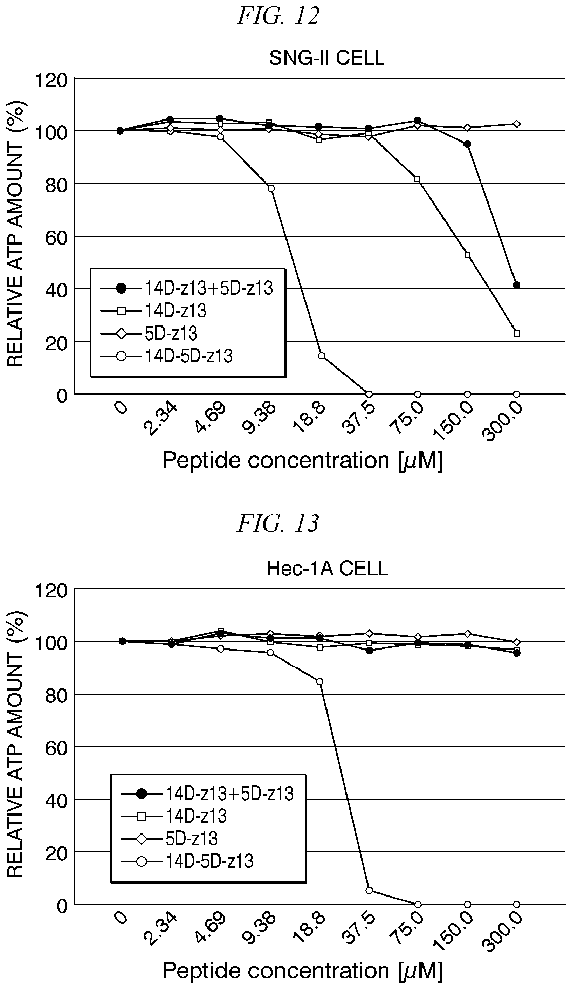

The cytocidal activity of each peptide was evaluated in the same manner as that of Example 1, except that the final concentration of each peptide added to each cell disseminated into the 96-well plate was set to be 0 (peptide non-added), 2.34, 4.69, 9.38, 18.8, 37.5, 75.0, 150.0, or 300.0 µM. A relative fluorescence intensity (%) of each reaction solution in a case where the fluorescence intensity (RLU) of the reaction solution to which no peptide was added was set to 100% was calculated as a relative ATP amount (%). The calculation result is shown in FIGS. 10 to 14. In the figure, the "14D - Z13 + 5D -Z13" represents a relative ATP amount of the reaction solution to which both of a peptide A2-1 and a peptide A2-2 were added, the "14D - Z13" represents a relative ATP amount of the reaction solution to which only the peptide A2-1 was added, the "5D - Z13" represents a relative ATP amount of the reaction solution to which only the peptide A2-2 was added, and the "14D - 5D - Z13" represents a relative ATP amount (%) of the reaction solution to which only the peptide A2 was added, respectively. As shown in FIGS. 10 to 14, it was confirmed that the peptide A2 has cytocidal activity to not only the A431-CNGB3-myc cells but also various cell strains derived from a uterine cancer cell. In addition, in the cell treated with both of a peptide in which the KLAK peptide and the Z13 peptide were linked and a peptide in which the HLAH peptide and the Z13 peptide were linked, similar to the cell treated only with the peptide in which the KLAK peptide and the Z13 peptide were linked and the cell treated only with the peptide in which the HLAH peptide and the Z13 peptide were linked, the cytocidal activity was hardly observed or significantly weak.

### [Example 7]

Since the A431 cells are epithelial-like cell cancer-derived cell strains, the endometriosis model mouse in which the A431-CNGB3-myc cells were transplanted into the peritoneal cavity is a cancer-bearing mouse. Here, a peptide (IFLLWQR-RR-KLAKLAK-KLAKLAK-HLAHL, SEQ ID NO: 40) in which an effector peptide consisting of an amino acid sequence represented by SEQ ID NO: 1 and an IF7 peptide are linked (hereinafter, referred to as "IF7 (RR) - (K + H) peptide") was administered to the peritoneal cavity of the endometriosis model mouse, and the cytocidal activity to cancer cells was examined. In the IF7 (RR) - (K + H) peptide, the KLAK sequence and the HLAH sequence moiety were synthesized with D-amino acids, and other peptide moieties were synthesized with L-amino acids.

### <Tail vein administration of peptide>

50 µL of a solution obtained by dissolving the IF7 (RR) - (K + H) peptide in a physiological saline solution was administered into an endometriosis model mouse after 14 days from transplanting the A431-CNGB3-myc cells into the peritoneal cavity once a day for six consecutive days via a tail vein. The IF7 (RR) - (K + H) peptide was administered such that an administration amount per mouse weight was 0 µg/body (control), 10 µg/body, or 50 µg/body.

As a result, in the control group into which a peptide was not administered (n = 8), the number of dead individuals after 24 days from transplanting the A431-CNGB3-myc cells into the peritoneal cavity was 1, and the alive rate was 88.9%. With respect to this, in a group into which the IF7 (RR) - (K + H) peptide was administered such that the IF7 (RR) - (K + H) peptide was 10 µg/body/day or 50 µg/body (n = 6), the alive rate was 100%. In addition, at the time of the end of the test, the spread of cancer cells (A431-CNGB3-myc cells) in the peritoneal cavity of all mice was examined. In contrast, in the control group, the cancer cells relatively spread throughout the whole peritoneum, and in the group into which the IF7 (RR) - (K + H) peptide was administered, the spread of cancer cells in many mice was relatively limited. There was no particular change in the weight of each mouse regardless of the administration amount or the number of administrations of the IF7 (RR) - (K + H) peptide. From the result, it was recognized that the intravenously administered IF7 (RR) - (K + H) peptide has cytocidal activity to cancer cells and is useful as an anti-cancer drug.

### [Example 8]

The IF7 (RR) - (K + H) peptide used in Example 7 was intravenously administered to a cancer-bearing mouse in which a tumor was formed in the back, and cytocidal activity to cancer cells was examined.

### <Luciferase gene-transferred ovarian cancer cancer-bearing subcutaneous tumor mouse (OVCAR3-Luc mouse)>

As the tumor tissue to be transplanted into a mouse, a tumor tissue obtained by culturing a luciferase gene-introduced ovarian cancer cell strain (OVCAR3-Luc cells, transferred from another facility) was used. Culturing of OVCAR3-Luc cells was performed at 37°C in a 5 volume% carbon dioxide environment using RPMI medium 1640 (11875-093, gibe by life technologies) as a culture medium.

Approximately 1 × 10⁶ OVCAR3-Luc cells were transplanted into the back of an eight-week female SCID mouse (C.B-17/Icr-scid/scid Jcl family, supplied by CLEA Japan, Inc.) to prepare an OVCAR3-Luc mouse.

### <Tail vein administration of peptide>

In the same manner as that of Example 7, 50 µL of a solution obtained by dissolving the IF7 (RR) - (K + H) peptide in a physiological saline solution was administered into the OVCAR3-Luc mouse once a day for six consecutive days via a tail vein. The IF7 (RR) - (K + H) peptide was administered such that an administration amount per mouse weight was 0 µg/body (control) or 10 µg/body.

### <Measurement of size of tumor tissue>

Each mouse was subjected to a luminescence imaging test, and the number of photons of the tumors on the back and the size of the tumor tissue were measured over time.

### (1) Measurement of the number of photons

Measurement was performed using an in vivo luminescence imaging apparatus (Xenogen IVIS-200, manufactured by Caliper Corporation) using a luciferin-luciferase luminescence mechanism. First, 100 µL of a 30 mg/mL potassium D-luciferin (126-05116, manufactured by Wako Pure Chemical Industries, Ltd.) solution was administered to the peritoneal cavity of the OVCAR 3-Luc mouse. After 15 minutes from the administration, the mouse was measured with the in vivo luminescence imaging apparatus, and the number of photons was counted.

### (2) Measurement of tumor volume

The estimated tumor volume (mm³) on the back of each OVCAR3-Luc mouse was obtained from the long diameter of the tumor by the following equation. The long diameter (mm) and the short diameter (mm) of the tumor were measured using a caliper. 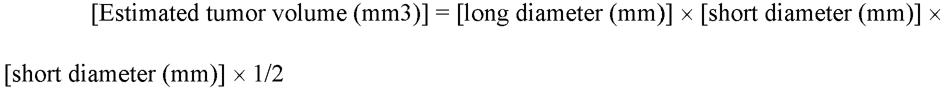 [Estimated tumor volume (mm3)] = [long diameter (mm)] × [short diameter (mm)] × [short diameter (mm)] × 1/2

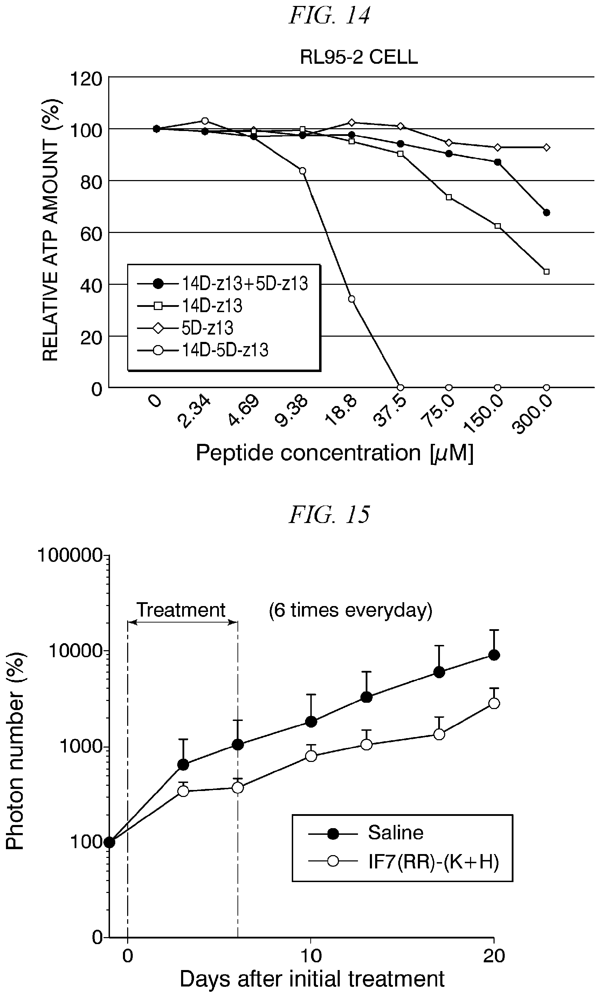

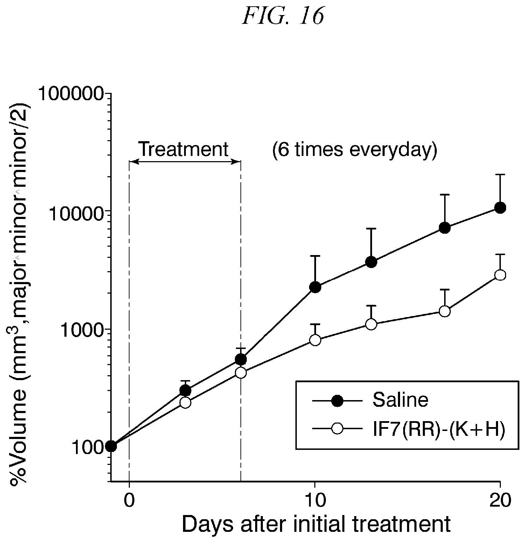

FIG. 15 shows a temporal change in an increase rate (%) in the number of the photons of the tumor on the back of each mouse, and FIG. 16 shows a temporal change in an increase rate (%) of the tumor volume (mm³) on the back of each mouse. Both of the increase rate of the number of the photons and the increase rate of the tumor volume were based on the value of the peptide solution on the day before the administration starting date (100%). In the figure, the "treatment" represents a treatment period in which the peptide solution was intravenously administered. As shown in FIG. 15, in the mouse treated with the IF7 (RR) - (K + H) peptide, the number of the photons was hardly increased during the peptide treatment period, and the increase rate after the peptide treatment period was also smaller than the control group (Saline). In addition, although there was no difference in the tumor volume between the control group and the IF7 (RR) - (K + H) peptide administration group during the peptide treatment period, the increase rate after the treatment period of the IF7 (RR) - (K + H) peptide administration group was apparently smaller. From the result, it can be said that administration of the IF7 (RR) - (K + H) peptide can kill the tumor tissue in vivo, and the peptide is useful as an anti-cancer drug.

### [Reference Example 2]

Paraffin-embedded sections of cancer tissues of various organs were immunostained with an anti-CNGB3 antibody to examine presence or absence of the expression of CNGB3.

As the tissue sections, two types (OV20811 and OV2088) among four types of commercially available human ovarian cancer tissue array (Ovary cancer tissue array (manufactured by US Biomax) and two types (FDA800a and MC964a) among cancer tissue arrays (Multiple organ tumor tissue array) of various organs were used. In addition, as the anti-CNGB3 antibody, among commercially available antibodies, two types of Biorbyt (Catalog No. orb 156415 BRT 100UG) (hereinafter, referred to as "antibody orb" sometimes) and Osenses (Code; OSC00253W) (hereinafter, referred to as "antibody 253W" were used).

Immunostaining of tissue sections was performed as follows. First, a paraffin-embedded tissue section was reacted with a blocking agent for liquid immunity test (Immunoblock, manufactured by DS Pharma Biomedical Co., Ltd.) for 30 minutes to block nonspecific reaction, and then washing treatment was performed twice with TBST (Tris buffer physiological saline containing Tween 20) for 5 minutes. Then, the tissue section was immersed in 0.3% hydrogen peroxide water, reacted for 5 minutes, blocked for endogenous peroxidase activity, and washed twice for 5 minutes with TBST. Subsequently, the tissue section was immersed in a 0.3% hydrogen peroxide solution, reacted for 5 minutes, and then subjected to blocking treatment of internal peroxidase activity. Thereafter, washing treatment was performed with TBST twice for 5 minutes. Subsequently, the tissue section was immersed in a primary antibody solution obtained by diluting the anti-CNGB3 antibody 100 times with REAL Antibody Diluent (Code S2022, manufactured by Dako Corporation) and reacted at room temperature for 30 minutes, and then washing treatment was performed twice with TBST for 5 minutes. In addition, the tissue section was reacted with a labeled secondary antibody (EnVision + System-HRP-labeled Polymer anti-rabbit antibody, manufactured by Dako Corporation) for 30 minutes at room temperature, and then washing treatment was performed twice with TBST for 5 minutes. Thereafter, the tissue section was treated with a DAB coloring reagent [DAB + Liquid (large size), RUO (K3468), manufactured by Dako Corporation] for 5 minutes to develop color. The tissue section after DAB coloring was washed with water, treated with Mayer's hematoxylin for 2 minutes to stain nuclei, and then sealed.

The staining result of OV20811 among human ovarian cancer tissue arrays is shown in Tables 5 to 10, and the staining result of OV2088 is shown in Tables 11 to 16, respectively. The staining result of FDA800a among cancer tissue arrays of various organs is shown in Tables 17 and 18, and the staining result of MC964a is shown in Tables 19 to 21, respectively. In the tables, in the column of each anti-CNGB3 antibody, "+" means the result of immunostaining with the antibody, and "-" means the result of no immunostaining with the antibody, respectively. It was recognized that most tissue sections were immunostained with the anti-CNGB3 antibody and CNGB3 was expressed.

**[Table 5]**

| OV20811 | | Grade | Stage | orb | 253W |
|---|---|---|---|---|---|
| 1 | Serous adenocarcinoma | 1 | Ia | + | + |
| 2 | Serous adenocarcinoma | | I | - | - |
| 3 | Adenocarcinoma (sparse) | - | Ia | + | + |
| 4 | Serous papillary adenocarcinoma | 2 | II | + | + |
| 5 | Serous papillary adenocarcinoma | | Ia | - | + |
| 6 | Mucinous papillary adenocarcinoma | 1 | Ic | - | + |
| 7 | Mucinous papillary adenocarcinoma (fibrous tissue and blood vessel) | - | Ia | + | - |
| 8 | Mucinous papillary adenocarcinoma | 1 | | + | - |
| 9 | | | | | |
| 10 | | | | + | + |
| 11 | | | IIa | + | + |
| 12 | Serous papillary adenocarcinoma | 2 | Ic | + | + |
| 13 | | | Ib | + | - |
| 14 | | | IIIc | + | + |
| 15 | | | | + | |
| 16 | | | IV | + | - |
| 17 | | 3 | Ic | + | + |
| 18 | Serous papillary adenocarcinoma (carcinoma sparse necrosis) | - | IIIa | + | + |
| 19 | Serous papillary adenocarcinoma | 3 | Ic | + | + |
| 20 | | | Ib | + | + |
| 21 | | 2 | | - | + |
| 22 | | | IIb | + | + |
| 23 | | | Ia | + | + |
| 24 | | 3 | IIb | + | + |
| 25 | | | Ib | + | + |
| 26 | Mucinous papillary adenocarcinoma | | | + | - |
| 27 | | | Ia | + | + |
| 28 | | | IIa | + | + |
| 29 | | | Ib | + | + |
| 30 | Serous papillary adenocarcinoma | 2 | Ia | + | + |
| 31 | | | IIa | + | + |
| 32 | | - | Ib | + | + |
| 33 | | 3 | Ia | + | + |
| 34 | | 2 | IIIc | + | + |
| 35 | | 2-3 | II | + | + |
| 36 | Mucinous papillary adenocarcinoma | 2 | I | + | + |
| 37 | Mucinous papillary adenocarcinoma (tumoral necrosis) | - | Ib | - | - |

**[Table 6]**

| OV20811 | | Grade | Stage | orb | 253W |
|---|---|---|---|---|---|
| 38 | Serous papillary adenocarcinoma | 3 | IIa | + | + |
| 39 | | | IV | + | + |
| 40 | | | | + | + |
| 41 | | 2 | Ib | + | + |
| 42 | | 2-3 | Ia | - | + |
| 43 | Serous papillary adenocarcinoma (fibrous tissue and blood vessel) | - | IIIc | + | + |
| 44 | Serous papillary adenocarcinoma | 3 | IV | + | + |
| 45 | Mucinous papillary adenocarcinoma | | IIIc | + | + |
| 46 | Serous papillary adenocarcinoma | | Ia | + | + |
| 47 | | | | + | + |
| 48 | | - | | + | + |
| 49 | | 1 | IIIb | + | + |
| 50 | | 3 | Ia | + | + |
| 51 | | | IIb | + | + |
| 52 | Serous adenocarcinoma | 2 | II | + | + |
| 53 | Serous papillary adenocarcinoma | 3 | Ia | + | + |
| 54 | | 2 | | + | + |
| 55 | | 3 | IIa | + | + |
| 56 | Mucinous papillary adenocarcinoma | | Ia | + | + |
| 57 | Serous papillary adenocarcinoma | | II | + | + |
| 58 | Serous adenocarcinoma | 2 | IIIc | + | + |
| 59 | Serous papillary adenocarcinoma | 3 | Ib | + | + |
| 60 | Serous papillary adenocarcinoma | | IIIc | + | + |
| 61 | Mucinous papillary adenocarcinoma with necrosis | | Ia | + | + |
| 62 | Serous papillary adenocarcinoma | | IIa | + | + |
| 63 | | | Ia | + | + |
| 64 | | - | IIa | + | + |
| 65 | Serous adenocarcinoma | 3 | Ia | + | + |
| 66 | Serous papillary adenocarcinoma | | | + | + |
| 67 | | | II | + | + |
| 68 | | | Ib | + | + |
| 69 | | | Ic | + | + |
| 70 | | 2 | Ia | + | + |
| 71 | Mucinous papillary adenocarcinoma | 3 | | + | + |
| 72 | Mucinous papillary adenocarcinoma (corpus albicans tissue) | - | Ib | + | + |
| 73 | Serous papillary adenocarcinoma (fibrous tissue) | - | Ia | + | + |
| 74 | Serous papillary adenocarcinoma | 3 | III | + | + |

**[Table 7]**

| OV20811 | | Grade | Stage | orb | 253W |
|---|---|---|---|---|---|
| 75 | Serous papillary adenocarcinoma (ovary tisuue) | - | Ic | - | + |
| 76 | Serous papillary adenocarcinoma | 3 | IIa | + | + |
| 77 | | | | + | + |
| 78 | | | IIb | + | + |
| 79 | Mucinous papillary adenocarcinoma with necrosis | | II | + | + |
| 80 | Serous papillary adenocarcinoma | | Ib | + | + |
| 81 | | | | + | + |
| 82 | Serous papillary adenocarcinoma (ovary tisuue) | - | II | + | + |
| 83 | Serous papillary adenocarcinoma | 3 | Ia | + | + |
| 84 | Serous papillary adenocarcinoma (chronic inflammation of fibrous tissue and blood vessel) | - | | + | + |
| 85 | Serous papillary adenocarcinoma | 3 | Ib | + | + |
| 86 | | | I | + | + |
| 87 | | | Ia | + | + |
| 88 | Serous papillary adenocarcinoma with necrosis | | I | + | + |
| 89 | Serous papillary adenocarcinoma | 2 | | + | + |
| 90 | | 3 | II | + | + |
| 91 | | | Ic | - | + |
| 92 | Mucinous papillary adenocarcinoma with necrosis | | Ib | + | + |
| 93 | Adenocarcinoma (sparse) | - | III | - | + |
| 94 | Serous papillary adenocarcinoma | 3 | I | + | + |
| 95 | | | Ia | + | + |
| 96 | Mucinous papillary adenocarcinoma | | Ib | + | + |
| 97 | Mucinous adenocarcinoma | | IIa | | + |
| 89 | | | Ib | + | + |
| 99 | Adenocarcinoma | | Ia | + | + |
| 100 | | | II | + | + |
| 101 | | 2 | Ia | + | + |
| 102 | Endometrioid adenocarcinoma | 1 | Ib | + | + |
| 103 | Endometrioid adenocarcinoma (tumoral necrosis) | - | II | - | - |
| 104 | Endometrioid adenocarcinoma | 1 | | + | + |
| 105 | Endometrioid adenocarcinoma (tumoral necrosis) | - | Ic | - | - |

**[Table 8]**

| OV20811 | | Grade | Stage | orb | 253W |
|---|---|---|---|---|---|
| 106 | Endometrioid adenocarcinoma | 1 | Ia | + | + |
| 107 | Endometrioid adenocarcinoma (sparse) | | IIa | - | + |
| 108 | Endometrioid adenocarcinoma | | Ia | + | + |
| 109 | | | I | - | + |
| 110 | | | | + | + |
| 111 | | | Ib | + | + |
| 112 | | | Ia | + | + |
| 113 | | | IIa | + | + |
| 114 | | | I | + | + |
| 115 | | | Ia | + | + |
| 116 | | 3 | I | + | + |
| 117 | | 2 | Ia | + | + |
| 118 | | | I | + | + |
| 119 | | 3 | IIa | + | + |
| 120 | | | IV | + | + |
| 121 | | 2 | Ic | + | + |
| 122 | | | IIc | + | + |
| 123 | | 3 | Ia | + | + |
| 124 | | | Ic | + | + |
| 125 | | | Ib | + | + |
| 126 | | | IIa | + | + |
| 127 | Clear cell carcinoma | - | I | | |
| 128 | | | | + | + |
| 129 | Clear cell carcinoma (sparse) with massive necrosis | | IIIc | - | + |
| 130 | Clear cell carcinoma (sparse) with necrosis | | I | + | + |
| 131 | Clear cell carcinoma | | | + | + |
| 132 | | | | + | + |
| 133 | Clear cell carcinoma (tumoral necrosis) | | II | - | - |
| 134 | Clear cell carcinoma | | Ic | + | + |
| 135 | | | I | + | + |
| 136 | | | | + | + |
| 137 | Undifferentiated carcinoma | | | + | + |
| 138 | | | Ia | + | + |
| 139 | Squamous cell carcinoma from malignant transformation of teratoma | 1 | I | + | + |
| 140 | | 2 | Ia | + | + |
| 141 | | | IIa | + | + |
| 142 | | | IIIc | + | + |
| 143 | | | Ia | + | + |
| 144 | | 3 | | + | + |

**[Table 9]**

| OV20811 | | Grade | Stage | orb | 253W |
|---|---|---|---|---|---|
| 145 | Squamous cell carcinoma | | Ib | + | + |
| 146 | Transitional cell carcinoma | | | + | + |
| 147 | | | IIIc | + | + |
| 148 | | | Ia | - | + |
| 149 | | | | + | + |
| 150 | Endodermal sinus carcinoma | | | - | + |
| 151 | | | IV | - | + |
| 152 | | | Ic | - | - |
| 153 | | | | - | + |
| 154 | | | Ib | - | + |
| 155 | | | I | - | + |
| 156 | | | | - | + |
| 157 | | | II | + | + |
| 158 | | | Ib | - | + |
| 159 | | | II | - | + |
| 160 | | | Ib | - | + |
| 161 | | - | IV | - | - |
| 162 | | | Ia | + | - |
| 163 | | | | + | - |
| 164 | Granular cell tumor | | I | - | + |
| 165 | | | Ic | - | + |
| 166 | | | Ib | - | - |
| 167 | | | I | - | + |
| 168 | | | Ia | - | + |
| 169 | | | Ib | - | - |
| 170 | | | Ia | - | + |
| 171 | | | IIb | + | - |
| 172 | | | Ic | - | - |
| 173 | | | I | - | + |
| 174 | | | Ia | - | - |
| 175 | | | | - | - |
| 176 | | | | - | + |
| 177 | | | | + | + |
| 178 | | | | - | - |

**[Table 10]**

| OV20811 | | Grade | Stage | orb | 253W |
|---|---|---|---|---|---|
| 179 | Granular cell tumor | | Ib | + | - |
| 180 | | | | - | - |
| 181 | | | Ia | - | - |
| 182 | | | II | - | + |
| 183 | | | Ib | N.T. | + |
| 184 | Dysgerminoma | | Ia | + | + |
| 185 | | | Ib | + | + |
| 186 | | | Ia | + | + |
| 187 | | | | + | + |
| 188 | | | | + | - |
| 189 | | | IIb | + | - |
| 190 | | | Ia | - | - |
| 191 | | | I | - | + |
| 192 | | | Ia | - | - |
| 193 | | - | | + | + |
| 194 | Interstitial cell tumor | | | - | - |
| 195 | Sertoli cell tumor | | | + | + |
| 196 | | | Ic | - | - |
| 197 | | | I | - | - |
| 198 | Theca cell tumor | | Ia | - | - |
| 199 | | | | - | - |
| 200 | Malignant theca cell tumor | | Ib | - | + |
| 201 | | | III | - | - |
| 202 | Malignant teratoma | | Ia | - | + |
| 203 | Immature teratoma | | Ib | - | - |
| 204 | | | | - | - |
| 205 | | | I | - | - |
| 206 | Gynandroblastoma | | Ia | - | - |
| 207 | Malignant fibroepithelioma | | | - | - |
| 208 | Malignant non-specificity steroid cell tumor | | | - | - |

**[Table 11]**

| OV2088 | | Grade | Stage | orb | 253W |
|---|---|---|---|---|---|
| 1 | Serous papillary adenocarcinoma | 1 | Ia | + | + |
| 2 | | | | + | + |
| 3 | | 2 | Ib | + | + |
| 4 | | | | - | - |
| 5 | | 1 | Ia | | + |
| 6 | | | | - | + |
| 7 | | 2 | Ib | + | + |
| 8 | | | | + | + |
| 9 | | 1 | Ia | - | - |
| 10 | | | | - | - |
| 11 | | | | - | + |
| 12 | | | | - | + |
| 13 | | | | - | - |
| 14 | Serous papillary adenocarcinoma (fibrous tissue and blood vessel) | | | | |
| 15 | Serous papillary adenocarcinoma | 1 | Ic | | |
| 16 | | | | | |
| 17 | Serous adenocarcinoma | 2 | I | + | + |
| 18 | | | | + | + |
| 19 | Serous papillary adenocarcinoma | 1 | | + | + |
| 20 | | | | - | + |
| 21 | | | Ia | - | + |
| 22 | | | | + | - |
| 23 | | 2 | Ic | - | + |
| 24 | | | | + | + |
| 25 | | | III | - | - |
| 26 | | | | | |
| 27 | | | Ia | | + |
| 28 | | | | - | + |
| 29 | | | | - | + |
| 30 | | | | - | - |
| 31 | | | | - | - |
| 32 | | | | - | - |
| 33 | | | Ib | + | + |
| 34 | | | | + | + |
| 35 | Serous adenocarcinoma with necrosis | | IIIa | - | - |
| 36 | Serous adenocarcinoma (sparse) | | | + | - |
| 37 | Serous papillary adenocarcinoma | | I | + | + |
| 38 | | | | + | + |
| 39 | | | Ib | + | + |
| 40 | | | | + | - |

**[Table 12]**

| OV2088 | | Grade | Stage | orb | 253W |
|---|---|---|---|---|---|
| 41 | Serous adenocarcinoma (sparse) | 2 | Ib | - | - |
| 42 | Serous adenocarcinoma (fibrous tissue and blood vessel) | - | | - | - |
| 43 | Serous adenocarcinoma | 3 | Ia | - | - |
| 44 | | | | - | - |
| 45 | | 2 | | - | + |
| 46 | | | | - | + |
| 47 | Serous adenocarcinoma (fibrous tissue and blood vessel) | - | | | |
| 48 | | | | - | - |
| 49 | Serous adenocarcinoma | 3 | Ib | + | + |
| 50 | | | | + | + |
| 51 | Serous adenocarcinoma (ovarian tissue) | - | | + | + |
| 52 | | | | | + |
| 53 | Serous papillary adenocarcinoma | 3 | Ia | - | + |
| 54 | | | | - | + |
| 55 | | 2 | II | + | + |
| 56 | | | | + | + |
| 57 | | 3 | Ib | - | + |
| 58 | | | | | + |
| 59 | | 2 | | + | + |
| 60 | | | | + | - |
| 61 | | | III | - | - |
| 62 | | | | - | - |
| 63 | | | I | + | + |
| 64 | | | | - | + |
| 65 | Serous adenocarcinoma (necrotic tissue) | - | | | |
| 66 | | | | | |
| 67 | Serous adenocarcinoma | 3 | Ib | - | + |
| 68 | | | | + | + |
| 69 | | | | + | + |
| 70 | Serous papillary adenocarcinoma (fibrous tissue and blood vessel) | - | | + | + |
| 71 | Serous adenocarcinoma (necrotic tissue) | | Ic | - | + |
| 72 | Serous adenocarcinoma | 3 | | - | + |
| 73 | | | III | - | + |
| 74 | | | | - | - |
| 75 | | | II | + | + |
| 76 | | | | + | + |
| 77 | | | Ic | - | + |
| 78 | | | | - | + |
| 79 | | | Ia | - | + |
| 80 | | | | - | + |

**[Table 13]**

| OV2088 | | Grade | Stage | orb | 253W |
|---|---|---|---|---|---|
| 81 | Serous adenocarcinoma | 3 | IIc | + | + |
| 82 | | | | + | + |
| 83 | | | Ia | + | + |
| 84 | | | | - | + |
| 85 | | 2 | Ib | + | + |
| 86 | Serous adenocarcinoma (ovarian tissue) | - | | - | + |
| 87 | Serous adenocarcinoma | 3 | II | + | + |
| 88 | | | | + | + |
| 89 | | | I | - | + |
| 90 | | | | - | + |
| 91 | | | II | - | + |
| 92 | | | | - | + |
| 93 | | | IV | + | - |
| 94 | | | | + | - |
| 95 | | | II | - | + |
| 96 | | | | - | + |
| 97 | | | IIa | | + |
| 89 | | | | | + |
| 99 | | | Ia | - | - |
| 100 | | | | + | + |
| 101 | Serous adenocarcinoma (chronic inflammation of fibrous tissue and blood vessel) | - | | + | + |
| 102 | Serous adenocarcinoma | 3 | | + | + |
| 103 | Serous adenocarcinoma (fibrous tissue and blood vessel) | - | Ic | + | + |
| 104 | | | | | + |
| 105 | Serous adenocarcinoma | 3 | Ic | + | + |
| 106 | | | | | |
| 107 | Serous papillary adenocarcinoma | | Ib | - | - |
| 108 | | | | | |
| 109 | Serous adenocarcinoma | | Ia | - | - |
| 110 | | | | - | - |
| 111 | | | Ib | - | + |
| 112 | Serous adenocarcinoma (ovarian tissue) | - | | - | + |
| 113 | - Serous adenocarcinoma | 3 | Ia | + | - |
| 114 | | | | + | - |
| 115 | | | I | - | - |
| 116 | | | | | + |
| 117 | | | | | |
| 118 | | | | - | + |
| 119 | | | | - | + |
| 120 | | | | - | - |

**[Table 14]**

| OV2088 | | Grade | Stage | orb | 253W |
|---|---|---|---|---|---|
| 121 | Serous adenocarcinoma with clear cell carcinoma | 3 | Ia | | + |
| 122 | | | | | |
| 123 | Mucinous papillary adenocarcinoma | 1 | I | - | - |
| 124 | | | | - | - |
| 125 | | 2 | Ic | - | - |
| 126 | | | | - | + |
| 127 | Serous papillary adenocarcinoma | 1-2 | II | - | - |
| 128 | | | | | |
| 129 | Mucinous papillary adenocarcinoma | 1 | I | + | + |
| 130 | | | | + | + |
| 131 | | | Ia | + | + |
| 132 | | | | + | + |
| 133 | | 1-2 | | + | + |
| 134 | | | | + | + |
| 135 | Mucinous adenocarcinoma | 2 | | - | - |
| 136 | | | | | |
| 137 | | | I | | |
| 138 | | | | - | - |
| 139 | Adenocarcinoma | 3 | Ia | - | - |
| 140 | | | | | |
| 141 | Adenocarcinoma from malignant transformation of teratoma | | | - | - |
| 142 | | | | - | - |
| 143 | Adenocarcinoma | | | - | - |
| 144 | | | | - | - |
| 145 | Endometrioid carcinoma (sparse) | | Ib | + | + |
| 146 | | | | + | + |
| 147 | Squamous cell carcinoma from malignant transformation of teratoma (mature teratoma sparse) | | IIIc | N.T. | N.T. |
| 148 | | | | + | + |
| 149 | Squamous celll carcinoma from malignant transformation of teratoma | 3 | Ib | + | - |
| 150 | | | | - | - |
| 151 | Squamous celll carcinoma | | | | + |
| 152 | | | | | + |
| 153 | Squamous cell carcinoma from malignant transformation of teratoma | | Ia | + | - |
| 154 | | | | + | - |
| 155 | Endodermal sinus carcinoma | - | | - | + |
| 156 | | | | - | + |
| 157 | | | Ib | | + |
| 158 | | | | - | + |
| 159 | | | | - | - |
| 160 | | | | - | - |

**[Table 15]**

| OV2088 | | Grade | Stage | orb | 253W |
|---|---|---|---|---|---|
| 161 | Endometrioid carcinoma | 2 | Ia | + | + |
| 162 | | | | + | + |
| 163 | Granular cell tumor | - | | - | - |
| 164 | | | | - | + |
| 165 | | | | + | + |
| 166 | | | | + | + |
| 167 | | | Ib | | |
| 168 | | | | + | + |
| 169 | | | | - | - |
| 170 | | | | - | - |
| 171 | Granulosa-theca cell tumor | | | | |
| 172 | | | | | |
| 173 | Granular cell tumor | | | - | - |
| 174 | | | | - | - |
| 175 | | | Ia | - | + |
| 176 | | | | - | + |
| 177 | | | | + | - |
| 178 | | | | + | - |
| 179 | Clear cell carcinoma | 2 | I | + | + |
| 180 | | | | + | + |
| 181 | | 3 | | + | + |
| 182 | | | | + | + |
| 183 | Clear cell carcinoma with necrosis | 2 | IIIc | + | + |
| 184 | | | | | |
| 185 | Dysgerminoma | | I | - | + |
| 186 | | | | - | + |
| 187 | | | Ia | + | + |
| 188 | | | | + | + |
| 189 | | | Ib | - | - |
| 190 | | | | - | - |
| 191 | Immature teratoma | | | - | + |
| 192 | | | | - | - |
| 193 | | | Ia | - | + |
| 194 | | | | - | - |
| 195 | Strumal carcinoid (sparse) | | Ib | + | + |
| 196 | | | | + | + |
| 197 | Undifferentiated carcinoma with neruoendocrine feature | | | + | |
| 198 | | | | + | - |
| 199 | Transitional cell carcinoma | 2 | | + | + |
| 200 | | | | + | N.T. |

**[Table 16]**

| OV2088 | | Grade | Stage | orb | 253W |
|---|---|---|---|---|---|
| 201 | Carcinosarcoma | | Ia | - | - |
| 202 | | | | - | - |
| 203 | Cellular fibroma | | - | - | - |
| 204 | | | | - | - |
| 205 | Embryonal carcinoma | - | Ia | - | - |
| 206 | | | | N.T. | N.T. |
| 207 | Diffuse B-cell lymphoma | | - | - | - |
| 208 | | | | - | - |

**[Table 17]**

| FDA800a | | | orb | 253W |
|---|---|---|---|---|
| 1 | Skin | Basal cell carcinoma of occipitalia skin | + | - |
| 2 | | Squamous cell carcinoma of left chest wall | + | - |
| 3 | Lung | Small cell carcinoma | - | - |
| 4 | | Adenocarcinoma | + | - |
| 5 | | Squamous cell carcinoma | - | + |
| 6 | | Large cell carcinoma | + | + |
| 7 | | Bronchoalveolar carcinoma | - | - |
| 8 | Testis | Spermatocytoma | + | - |
| 9 | | Spermatocytoma | - | - |
| 10 | | Embryonal carcinoma | + | + |
| 11 | Prostate | Adenocarcinoma (Gleason grade:4; Gleason score:4+5) | + | + |
| 12 | | Rhabdomyosarcoma | - | - |
| 13 | Gall bladder | Adenocarcinoma | + | + |
| 14 | | Adenosquamous carciinoma | + | - |
| 15 | Small intestine | Adenocarcinoma | + | - |
| 16 | | Low garde malignant interstitialoma | - | + |
| 17 | Pancreas | Duct Adenocarcinoma | + | + |
| 18 | | Islet cell carcinoma | - | + |
| 19 | Colon | Adenocarcinoma | + | + |
| 20 | | Low garde malignant interstitialoma | + | + |
| 21 | Rectum | Adenocarcinoma | + | + |
| 22 | | Low garde malignant interstitialoma | - | - |
| 23 | | Malignant melanoma | + | + |
| 24 | Esophagus | Squamous cell carcinoma | + | + |
| 25 | | Adenocarcinoma | + | - |
| 26 | Appendix | Adenocarcinoma | + | + |
| 27 | Tongue | Squamous cell carcinoma | + | + |
| 28 | Parotid gland | Adenoid cystic carcinoma | + | + |
| 29 | Stomach | Mucinous adenocarcinoma | + | + |
| 30 | Liver | Hepatoblastoma | + | - |
| 31 | | Hepatocellular carcinoma | + | + |
| 32 | Breast | Ductal carcinoma in situ | + | + |
| 33 | | Invasive ductal carcinoma | + | + |
| 34 | | Duvtal carcinoma in situ | + | - |
| 35 | | Medullary carcinoma | N.T. | N.T. |
| 36 | Uterus | Adenocarcinoma endometrium | + | + |
| 37 | | Clear cell carcinoma with necrosis (sparse) | + | + |
| 38 | | Leiomyoma | - | - |
| 39 | | Squamous cell carcinoma | + | + |
| 40 | Cervix | Endocervical type adenocarcinoma | + | + |

**[Table 18]**

| FDA800a | | | orb | 253W |
|---|---|---|---|---|
| 41 | Ovary | Mucinous adenocarcinoma | - | + |
| 42 | | Serous adenocarcinoma | + | + |
| 43 | Mediastinum | Thymoma (type B3) | + | + |
| 44 | Thyroid | Medullary carcinoma | - | - |
| 45 | | Papilly carcinoma | + | + |
| 46 | Brain | Atypical meningioma | - | + |
| 47 | | Malignant ependymoma of right occipital lobe | - | - |
| 48 | | Anaplastic oligodendoroglioma with calcification (sparse) | - | - |
| 49 | | Pantmorphic glioblastoma of right occipital lobe | + | - |
| 50 | | Medulloblastoma of cerebullum | + | - |
| 51 | Bladder | Transitional cell carcinoma | - | - |
| 52 | | Low garde malignant leiomysarcoma | + | - |
| 53 | Kidney | Transitional cell carcinoma | - | + |
| 54 | | Papillary renal cell carcinoma | - | - |
| 55 | | Clear cell carcinoma | + | + |
| 56 | Lymph node | Diffuse B-cell lymphoma | + | - |
| 57 | | Diffuse B-cell lymphoma of right thigh | - | - |
| 58 | | Diffuse B-cell lymphoma of mandible | - | - |
| 59 | | Diffuse B-cell lymphoma of spleen | - | - |
| 60 | | Hodgkin's lymphoma of left clavicle | - | - |
| 61 | Soft tissue | Leiomyosarcoma of chest wall | - | - |
| 62 | | Embryonal rhabdomyosarcoma of left leg | - | - |
| 63 | Retroperitone um | Rhabdomyosarcoma | - | - |
| 64 | | Primitive neuroectodermal tumor | - | - |
| 65 | Abdominal wall | Clear cell sarcoma | + | + |
| 66 | Retroperitone um | Neuroblastoma | - | - |
| 67 | Peritoneal cavity | Mucinous liposarcoma | - | - |
| 68 | Pelvic cavity | Chordoma | + | + |
| 69 | Soft tissue | Neurofibroma of right low back | N.T. | N.T. |
| 70 | Pelvic cavity | Synovial sarcoma of right chest wall | - | - |
| 71 | Bone | Osteosarcoma of right femur | - | - |
| 72 | Peritoneum | Malignant mesothelioma | - | - |

**[Table 19]**

| MC964a | | | orb | 253W |
|---|---|---|---|---|
| 1 | Cerebrum | Astrocytoma | + | - |
| 2 | | Anaplastic oligogendroglioma | - | - |
| 3 | | Malignant ependymoma | + | - |
| 4 | | Astrocytoma | + | - |
| 5 | | Astrocytoma | + | - |
| 6 | | Glioblastoma | + | + |
| 7 | Esophagus | Squamous cell carcinoma (sparse) | + | + |
| 8 | | Squamous cell carcinoma (sparse) | + | + |
| 9 | | Squamous cell carcinoma | + | + |
| 10 | Stomach | Adenocarcinoma | - | - |
| 11 | | Adenocarcinoma | + | + |
| 12 | | Interstitialoma | + | + |
| 13 | Liver | Hepatocellular carcinoma | + | + |
| 14 | | Cholangioma | + | + |
| 15 | | Hepatoblastoma | + | + |
| 16 | Small intestine | Adenocarcinoma | + | + |
| 17 | | Diffuse B-cell lymphoma | - | - |
| 18 | | Interstitialoma | + | + |
| 19 | Colon | Adenocarcinoma | + | + |
| 20 | | Interstitialoma | - | + |
| 21 | | Diffuse B-cell lymphoma | - | - |
| 22 | Rectum | Adenocarcinoma | + | + |
| 23 | | Interstitialoma | + | + |
| 24 | | Interstitialoma | + | - |
| 25 | Pancreas | Islet cell carcinoma | + | + |
| 26 | | Adenocarcinoma | + | + |
| 27 | | Adenocarcinoma | + | + |
| 28 | Omentum majus | Metastatic adenocarcinoma | + | + |
| 29 | | Metastatic dysgerminoma | - | + |
| 30 | | Interstitialoma | - | - |
| 31 | Lung | Small cell undifferentiated carcinoma | - | - |
| 32 | | Small cell undifferentiated carcinoma | - | - |
| 33 | | Atypical carcinoid | + | - |
| 34 | | Squamous cell carcinoma | + | + |
| 35 | | Adenocarcinoma | + | + |
| 36 | | Squamous cell carcinoma | + | + |

**[Table 20]**

| MC964a | | | orb | 253W |
|---|---|---|---|---|
| 37 | Uterine cervix | Squamous cell carcinoma a | + | + |
| 38 | | Squamous cell carcinoma | + | + |
| 39 | | Squamous cell carcinoma | + | + |
| 40 | Uterus | Endometrioid adenocarcinoma | + | + |
| 41 | | Endometrioid adenocarcinoma | + | + |
| 42 | | Clear cell carcinoma | - | + |
| 43 | Breast | Invasive lobular carcinoma | + | - |
| 44 | | Invasive ductal carcinoma | - | + |
| 45 | | Cystosarcoma phyllodes | - | - |
| 46 | Ovary | Serous adenocarcinoma | - | - |
| 47 | | Serous adenocarcinoma | - | + |
| 48 | | Serous adenocarcinoma | + | + |
| 49 | Spleen | Diffuse B-cell lymphoma | + | - |
| 50 | | Diffuse non-Hodgkin's lymphoma | - | - |
| 51 | | Diffuse B-cell lymphoma | + | + |
| 52 | Prostate | Adenocarcinoma (Gleason grade:3; Gleason score:3+4) | + | + |
| 53 | | Adenocarcinoma (Gleason grade:3; Gleason score:3+3) | + | + |
| 54 | | Adenocarcinoma (Gleason grade:3; Gleason score:2+3) | - | + |
| 55 | Testis | Seminoma with necrosis | - | + |
| 56 | | Diffuse B-cell lymphoma | - | - |
| 57 | | Embryonal carcinoma | + | + |
| 58 | Kidney | Clear cell carcinoma | - | + |
| 59 | | Clear cell carcinoma | - | + |
| 60 | | Sarcomatoid carcinoma | - | - |
| 61 | Bladder | Transitional cell carcinoma | + | + |
| 62 | | Mucinous adenocarcinoma | + | + |
| 63 | | Leiomyosarcoma | + | + |

**[Table 21]**

| MC964a | | | orb | 253W |
|---|---|---|---|---|
| 64 | Lymph node | Hodgkin's lymphoma of neck | + | + |
| 65 | | Hodgkin's lymphoma of neck | - | + |
| 66 | | Diffuse B-cell lymphoma of left groin | - | - |
| 67 | | Diffuse B-cell lymphoma of light elbow joint | - | - |
| 68 | Mediastinu m | Diffuse T-cell lymphoma of mediastinum | - | - |
| 69 | Mesentery | Diffuse B-cell lymphoma of mesentery | + | - |
| 70 | Bone | Osteosarcoma of left leg | + | + |
| 71 | | Osteosarcoma of right femur | - | + |
| 72 | | Osteosarcoma of right femur | - | + |
| 73 | | Dedifferentiation chondrosarcoma of pars sacralis | - | - |
| 74 | | Well-differentiated chondrosarcoma of right femur | + | + |
| 75 | | Mesenchymal chondrosarcoma of pars sacralis | + | - |
| 76 | Skin | Squamous cell carcinoma of cheek | + | + |
| 77 | Tongue | Squamous cell carcinoma of lip | + | + |
| 78 | Nose | Squamous cell carcinoma of nose | + | + |
| 79 | Tongue | Squamous cell carcinoma of pharynx | + | + |
| 80 | Larynx | Squamous cell carcinoma of larynx | + | + |
| 81 | | Squamous cell carcinoma of larynx | + | + |
| 82 | Pharynx | Diffuse B-cell lymphoma of pharynx | - | - |
| 83 | Pharynx | Squamous cell carcinoma of larynx | + | + |
| 84 | Nose | Squamous cell carcinoma of nasopharynx | - | - |
| 85 | Tongue | Squamous cell carcinoma | + | + |
| 86 | | Embryonal rhabdomyosarcoma | - | + |
| 87 | | Mucoepidermoid carcinoma | + | + |
| 88 | Fatty tissue | Mucoid liposarcoma of left leg | + | + |
| 89 | Fibrous tissue | Isolated fibroma of chest wall | - | - |
| 90 | | Fibrosarcoma of left forearm | + | + |
| 91 | Thyroid | Medullary carcinoma | + | + |
| 92 | | Papillary carcinoma | + | + |
| 93 | | Diffuse B-cell lymphoma | - | - |
| 94 | Skin | Malignant melanoma of anus margin | + | + |
| 95 | | Basal cell carcinoma of scalp | - | - |
| 96 | | Squamous cell carcinoma of left chest wall | + | + |

### [Example 9]

The amount of CNGB3 in exosomes isolated from blood was measured to examine whether it is possible to distinguish a patient with a CNGB3 high expression disease from a person without a CNGB3 high expression disease. The following tests were performed with an approval of the Hamamatsu Medical University Institutional Review Board.

### <Serum sample>

Blood collected from two healthy persons (persons with no tumor, who have not been confirmed to be affected by any disease and are expected to be healthy) and patients from Hamamatsu Medical University School of Medicine (three patients with ovarian cancer, three patients with uterine cancer, three patients with breast cancer, one patient with cervical cancer, two patients with colon cancer, three patients with endometriosis, and one patient with uterine adenomyosis patient) was centrifuged and the obtained serum aliquots were stored at - 80°C. The patients with uterine adenomyosis are patients who have developed both of endometriosis and fibroids.

### <Exosome separation>

The exosome was isolated from the serum using Exosome Isolation Kit (manufactured by Fujifilm Wako Pure Chemical Industries, Ltd.) according to the manufacturer's instruction. Specifically, first, the serum was centrifuged at 1,000 × g at 4°C° for 20 minutes, and the supernatant was recovered. The collected supernatant was centrifuged at 10,000 × g at 4°C for 30 minutes, and the supernatant was transferred to a new tube. Subsequently, 1.0 mL of the supernatant was mixed with exosome capture beads with a rotator at 4°C for 180 minutes. Subsequently, after washing the capture beads, the exosome was eluted with 100 µL of an elution buffer and stored at -20°C.

### <Peptide synthesis>

As a biotinylated Z13 peptide (peptide consisting of L-amino acids) in which biotin bound to a Z13 peptide (VRRADNRPG: SEQ ID NO: 3), one synthesized by GenScript Inc. was used.

### <Avidin blotting>

An exosome isolated from 800 µL of serum was mixed with a concentrated Laemmli buffer to prepare a measurement sample. The measurement sample was isolated by SDS-PAGE and then transferred to a PVDF membrane. The PVDF membrane was blocked in 5% BSA-containing TBST (0.1 v/v% Tween-20) at room temperature for 60 minutes. The PVDF membrane after blotting was washed and incubated overnight at 4°C° in 1.0 µg/mL of biotinylated Z13 peptide-containing TBST. Thereafter, the membrane was washed with TBST, and subsequently incubated at room temperature for 60 minutes in 0.1 µg/mL TBST containing HRP (horseradish peroxidase)-binding avidin (manufactured by Thermo Fisher Scientific). The CNGB3 on the membrane was detected using a CCD camera system (manufactured by ATTO) and ECL prime (manufactured by GE Healthcare). Densitometry analysis was performed using an image analysis software "CS Analyzer 4" (manufactured by ATTO).

### <Statistical analysis>

All analysis was performed using a medial statistics software "GraphPad Prism 6" (manufactured by GraphPad Software Corporation).

The signal intensity of the band of CNGB 3 and the relative value thereof (relative value with the signal intensity of healthy person 1 being 1) obtained in the result of avidin blotting of each measurement sample are shown in Table 22. However, since breast cancer patients 1 and 2 could secure only a small amount of serum, exosomes isolated from 100 µL of serum were used, and a breast cancer patient 3 used exosomes isolated from 37.5 µL of serum. For this reason, the signal intensity of breast cancer patient samples was corrected by multiplying by the reciprocal of a serum use amount.

**[Table 22]**

| Subject sample | Signal intensity | Relative signal intensity |
|---|---|---|
| Healthy person 1 | 1082305 | 1.00 |
| Healthy person 2 | 1188517 | 1.10 |
| Ovarian cancer patient 1 | 4377691 | 4.04 |
| Ovarian cancer patient 2 | 3907260 | 3.61 |
| Ovarian cancer patient 3 | 3620987 | 3.35 |
| Uterine body cancer patient 1 | 2681313 | 2.48 |
| Uterine body cancer patient 2 | 2939504 | 2.72 |
| Uterine body cancer patient 3 | 14049406 | 12.98 |
| Breast cancer patient 1 | 18864576 | 17.43 |
| Breast cancer patient 2 | 41851784 | 38.67 |
| Breast cancer patient 3 | 20114133 | 18.58 |
| Uterine cervix cancer patient 1 | 3480924 | 3.22 |
| Large intestine cancer patient 1 | 6057912 | 5.60 |
| Large intestine cancer patient 2 | 2285673 | 2.11 |
| Endometriosis patient 1 | 13117100 | 12.75 |
| Endometriosis patient 2 | 4358049 | 4.24 |
| Endometriosis patient 3 | 3454806 | 3.36 |
| Uterine adenomyosis patient 1 | 5835780 | 5.67 |

As a result, in all the samples, a band of CNGB3 was detected, and it was confirmed that CNGB3 was contained in the exosomes. The CNGB3 amount of exosomes isolated from cancer patients, endometriosis patients and uterine adenomyosis patients was more than two times the exosomes isolated from healthy persons. From the result, it was confirmed that in exosomes isolated from the serum of patients with high expression of CNGB3 in disease-causing cells of the patients, such as tumor patients, endometriosis patients, and uterine adenomyosis patients, a significantly larger amount of CNGB3 is contained than in the exosomes isolated from serum, and therefore, by setting an appropriate reference value (cutoff value), it is possible to evaluate a possibility of onset of a CNGB3 high expression disease based on the amount of CNGB3 in the exosomes.

In addition, it was confirmed that CNGB3 was even present in the exosomes of the healthy persons, although in a very small amount. This is presumably because a molecule having a high binding property to CNGB3 such as CNGB3-binding peptide was used to detect CNGB3 of exosomes in serum with a very high sensitivity. Since it was possible to detect exosomes including CNGB3 released from an extremely small amount of cancer cells in the bodies of the healthy persons, it could be expected that it is also possible to detect not only an advanced cancer but also an early cancer even in a case where the exosomes in serum are set as specimens by using a CNGB3-binding peptide.

## Claims

1. A cytocidal agent comprising:
a peptide consisting of an amino acid sequence represented by SEQ ID NO: 1 and a peptide consisting of an amino acid sequence represented by SEQ ID NO: 2, the peptide consisting of an amino acid sequence represented by SEQ ID NO: 2 being directly linked to the C-terminal side of the peptide consisting of an amino acid sequence represented by SEQ ID NO: 1; or
a peptide consisting of an amino acid sequence represented by SEQ ID NO: 7 and a peptide consisting of an amino acid sequence represented by SEQ ID NO: 1, the peptide consisting of an amino acid sequence represented by SEQ ID NO: 1 being linked via RR to the C-terminal side of the peptide consisting of an amino acid sequence represented by SEQ ID NO: 7, wherein
the peptide consisting of an amino acid sequence represented by SEQ ID NO: 1 is an effector peptide in which a peptide having apoptosis-inducing activity and a peptide having endosomal escape activity are linked in tandem, and
the peptide having apoptosis-inducing activity consists of the amino acid sequence from the first to the 14th amino acids in the amino acid sequence of SEQ ID NO: 1, and the peptide having endosomal escape activity consists of the amino acid sequence from the 15th to the 19th amino acids in the amino acid sequence of SEQ ID NO: 1.

2. The cytocidal agent according to Claim 1,
wherein the peptide consisting of an amino acid sequence represented by SEQ ID NO: 1 is
a peptide exclusively consisting of L-amino acids,
a peptide in which, in the amino acid sequence represented by SEQ ID NO: 1, the first to the 14th amino acids are D-amino acids, and the 15th to 19th amino acids are L-amino acids,
a peptide in which, in the amino acid sequence represented by SEQ ID NO: 1, the first to 14th amino acids are L-amino acids, and the 15th to the 19th amino acids are D-amino acids, or
a peptide exclusively consisting of D-amino acids.

3. The cytocidal agent according to Claim 1 or 2 for use in the treatment of a disease resulting from abnormal proliferation of a cell, wherein the disease is endometriosis or cancer.

## Patentansprüche

1. Ein zytotoxisches Mittel, umfassend:
ein Peptid bestehend aus einer durch SEQ ID NO: 1 dargestellten Aminosäuresequenz; und
ein Peptid bestehend aus einer durch SEQ ID NO: 2 dargestellten Aminosäuresequenz, wobei das Peptid bestehend aus einer durch SEQ ID NO: 2 dargestellten Aminosäuresequenz direkt an die C-terminale Seite des Peptids bestehend aus einer durch SEQ ID NO: 1 dargestellten Aminosäuresequenz gebunden ist, oder
ein Peptid bestehend aus einer durch SEQ ID NO: 7 dargestellten Aminosäuresequenz und ein Peptid bestehend aus einer durch SEQ ID NO: 1 dargestellten Aminosäuresequenz, wobei das Peptid bestehend aus einer durch SEQ ID NO: 1 dargestellten Aminosäuresequenz über RR an die C-terminale Seite des Peptids bestehend aus einer durch SEQ ID NO: 7 dargestellten Aminosäuresequenz gebunden ist, wobei
das Peptid bestehend aus einer durch SEQ ID NO: 1 dargestellten Aminosäuresequenz ein Effektorpeptid ist, in dem ein Peptid mit Apoptoseinduzierender Wirkung und ein Peptid mit Endosomen-Flucht-Aktivität in Tandemanordnung miteinander verbunden sind, und das Peptid mit Apoptoseinduzierender Wirkung aus der Aminosäuresequenz von der ersten bis zur vierzehnten Aminosäure in der Aminosäuresequenz gemäß SEQ ID NO: 1 besteht und das Peptid mit Endosomen-Flucht-Aktivität aus der Aminosäuresequenz von der fünfzehnten bis zur neunzehnten Aminosäure in der Aminosäuresequenz gemäß SEQ ID NO: 1 besteht.

2. Das zytotoxische Mittel gemäß Anspruch 1, wobei das Peptid bestehend aus einer durch SEQ ID NO: 1 dargestellten Aminosäuresequenz
ein Peptid ausschließlich bestehend aus L-Aminosäuren,
ein Peptid, in dem in der durch SEQ ID NO: 1 dargestellten Aminosäuresequenz die erste bis zur vierzehnten Aminosäure D-Aminosäuren sind und die fünfzehnte bis neunzehnte Aminosäure L-Aminosäuren sind,
ein Peptid, in dem in der durch SEQ ID NO: 1 dargestellten Aminosäuresequenz die erste bis zur vierzehnten Aminosäure L-Aminosäuren sind und die fünfzehnte bis neunzehnte Aminosäure D-Aminosäuren sind, oder
ein Peptid ausschließlich bestehend aus D-Aminosäuren ist.

3. Das zytotoxische Mittel gemäß Anspruch 1 oder 2 zur Verwendung in der Behandlung einer Erkrankung resultierend aus anormaler Proliferation einer Zelle, wobei die Erkrankung Endometriose oder Krebs ist.

## Revendications

1. Agent cytocide comprenant :
un peptide consistant en une séquence d'acides aminés représentée par la SEQ ID NO : 1 et un peptide consistant en une séquence d'acides aminés représentée par la SEQ ID NO : 2, le peptide consistant en une séquence d'acides aminés représentée par la SEQ ID NO : 2 étant directement lié au côté C-terminal du peptide consistant en une séquence d'acides aminés représentée par la SEQ ID NO : 1 ; ou
un peptide consistant en une séquence d'acides aminés représentée par la SEQ ID NO : 7 et un peptide consistant en une séquence d'acides aminés représentée par la SEQ ID NO : 1, le peptide consistant en une séquence d'acides aminés représentée par la SEQ ID NO : 1 étant lié via RR au côté C-terminal du peptide consistant en une séquence d'acides aminés représentée par la SEQ ID NO : 7,
dans lequel
le peptide consistant en une séquence d'acides aminés représentée par la SEQ ID NO : 1 est un peptide effecteur dans lequel un peptide ayant une activité d'induction de l'apoptose et un peptide ayant une activité d'échappement endosomal sont liés en tandem, et
le peptide ayant une activité d'induction de l'apoptose consiste en la séquence d'acides aminés ayant des premier au 14ème acides aminés dans la séquence d'acides aminés de la SEQ ID NO : 1, et le peptide ayant une activité d'échappement endosomal consiste en la séquence d'acides aminés allant des 15ème à 19ème acides aminés dans la séquence d'acides aminés de la SEQ ID NO : 1.

2. Agent cytocide selon la revendication 1,
dans lequel le peptide consistant en une séquence d'acides aminés représentée par la SEQ ID NO : 1 est
un peptide consistant exclusivement en acides L-aminés,
un peptide dans lequel, dans la séquence d'acides aminés représentée par la SEQ ID NO : 1, les premier à 14ème acides aminés sont des acides D-aminés, et les 15ème à 19ème acides aminés sont des acides L-aminés,
un peptide dans lequel, dans la séquence d'acides aminés représentée par la SEQ ID NO : 1, les premier à 14ème acides aminés sont des acides L-aminés, et les 15ème à 19ème acides aminés sont des acides D-aminés, ou
un peptide consistant exclusivement en acides D-aminés.

3. Agent cytocide selon la revendication 1 ou 2, pour une utilisation dans le traitement d'une maladie résultant d'une prolifération anormale d'une cellule, dans lequel la maladie est une endométriose ou un cancer.
